# EUROPÄISCHE PATENTSCHRIFT

(11) **EP 2 755 695 B1**
(45) Veröffentlichungstag und Bekanntmachung des Hinweises auf die Patenterteilung: **26.04.2017**
(21) Anmeldenummer: 12756213.0
(22) Anmeldetag: 10.09.2012
(51) Int. Cl.: A61L 2/24, A61G 9/02, A47L 15/42

(54) **REINIGUNGS- UND DESINFEKTIONSGERÄT ZUR BEHANDLUNG VON BEHÄLTERN FÜR MENSCHLICHE AUSSCHEIDUNGEN**
CLEANING AND DISINFECTING APPARATUS FOR TREATING CONTAINERS FOR HUMAN EXCRETIONS
APPAREIL DE NETTOYAGE ET DE DÉSINFECTION POUR LE TRAITEMENT DE RÉCIPIENTS POUR EXCRÉMENTS HUMAINS

(30) Priorität: 14.09.2011 DE 102011082654
(43) Veröffentlichungstag der Anmeldung: 23.07.2014
(73) Patentinhaber: MEIKO Maschinenbau GmbH & Co. KG, 77652 Offenburg (DE)
(72) Erfinder: SIMUNDIC, Marijan, 77797 Ohlsbach (DE); WIEGAND, Ingo, 77830 Bühlertal (DE); PEUKERT, Thomas, 77815 Bühl (DE)
(74) Vertreter: Herzog, Fiesser & Partner Patentanwälte PartG mbB
(86) Internationale Anmeldenummer: PCT/EP2012/067621
(87) Internationale Veröffentlichungsnummer: WO 2013/037723

(56) Entgegenhaltungen:
- EP-A2- 1 704 809
- WO-A2-2011/048575
- DE-A1- 3 628 793
- DE-A1- 10 048 081
- US-A1- 2012 138 092

## Beschreibung

### Gebiet der Erfindung

Die Erfindung betrifft ein Reinigungs- und Desinfektionsgerät sowie ein Verfahren zur Behandlung mindestens eines Behälters für menschliche Ausscheidungen. Derartige Reinigungs- und Desinfektionsgeräte und Verfahren werden beispielsweise im Krankenhaus- oder Pflegebereich eingesetzt, um Behälter wie beispielsweise Steckbecken, Urinflaschen, Bettpfannen, Nachtgeschirr, Nierenschalen, Waschschüsseln oder andere Behälter zu reinigen, welche zur Aufnahme menschlicher oder tierischer Ausscheidungen geeignet sind, insbesondere mit einem Volumen von mindestens 100 ml, beispielsweise 100-500 ml.

### Stand der Technik

Aus dem Stand der Technik sind eine Vielzahl von Reinigungsgeräten und Reinigungsverfahren zur Behandlung von Behältern für menschliche Ausscheidungen bekannt, Die zu reinigenden Behälter können größere Flüssigkeitsmengen oder Mengen an Feststoffabfällen enthalten, welche üblicherweise während der Reinigung entsorgt werden müssen. Herkömmliche Spülmaschinen sind somit für die Reinigung derartiger Behälter in der Regel nicht geeignet. Zudem können derartige Behälter infektiöse Abfälle enthalten oder anderweitig kontaminiert sein, sodass, neben einer Entleerung, auch üblicherweise eine Desinfektion erforderlich ist. Derartige Reinigungsgeräte zur Behandlung von Behältern für menschliche Ausscheidungen werden dementsprechend häufig auch als Reinigungs- und Desinfektionsgeräte (RDG) bezeichnet. Neben den genannten Behältern sind diese grundsätzlich auch zur Reinigung anderer medizinischer Gegenstände geeignet, wie sie beispielsweise in Krankenhäusern oder Altenpflegeeinrichtungen eingesetzt werden. Üblicherweise besteht das Reinigungsgut jedoch aus Urinflaschen, Steckbecken, Nierenschalen, Waschschüsseln oder ähnlichen Behältern, deren Reinigung die Entsorgung größerer Abfallmengen mit sich bringen kann.

DE 100 48 081 A1 beschreibt ein Verfahren zur Erkennung der Spülgutbeladung und/oder eines Verschmutzungsgrades von Spülgut in einer programmgesteuerten Geschirrspülmaschine. In der programmgesteuerten Geschirrspülmaschine wird das Spülgut in Geschirrkörben und ggf. Besteckkörben oder dgl. im türverschließbaren Spülraum des Gerätes abgelegt. Ein oder mehrere Bilderkennungssysteme erfassen die Beladung des Spülbehälters nach Art und Menge und/oder die Verschmutzung des Spülgutes schon vor oder bei Spülbeginn automatisch. Von dem Ergebnis ausgehend kann rechnergesteuert ein folgendes Spülprogramm hinsichtlich einer optimalen wirtschaftlichen und energiesparenden Arbeitsweise des Gerätes abgeändert werden.

Ein Beispiel eines Reinigungs- und Desinfektionsgerätes ist in DE 103 48 344 A1 gezeigt. Auf den Aufbau dieses Reinigungs- und Desinfektionsgerätes kann exemplarisch grundsätzlich auch im Rahmen der vorliegenden Erfindung Bezug genommen werden, wobei das dargestellte Reinigungs- und Desinfektionsgerät erfindungsgemäß ergänzt werden kann. Auch andere Ausgestaltungen sind jedoch grundsätzlich möglich. Das in DE 103 48 344 A1 beschriebene Reinigungs- und Desinfektionsgerät beinhaltet eine Vorrichtung zur Rückkühlung von Reinigungsgut. Dabei erfolgt innerhalb einer Kammer ein Abspülen des Reinigungsguts, woran sich ein Vorreinigungsspülschritt anschließt. Danach wird das in der Kammer enthaltene Reinigungsgut mit einem einen Klarspülerzusatz enthaltenden Wasser endgereinigt, bevor ein Desinfektionsschritt des Reinigungsguts in der Kammer durch Einleitung von Wasserdampf in diese Kammer erfolgt. In die mit Wasserdampf befüllte Kammer wird zwangsweise Luft bei geschlossener Tür eingebracht, wodurch ein Niederschlag von Wasserdampf innerhalb der Kammer sowie eine Abkühlung und eine Trocknung des in der Kammer enthaltenen Reinigungsguts bewirkt wird.

In einem Reinigungs- und Desinfektionsgerät läuft somit üblicherweise zur Behandlung des Reinigungsguts ein Programm ab, das üblicherweise in einer Steuerung des Geräts hinterlegt ist. Nach der Beladung des RDGs wählt üblicherweise ein Anwender ein Reinigungsprogramm aus und startet dieses üblicherweise durch Betätigen einer entsprechenden Taste, beispielsweise an einer Folientastatur. Der Anwender muss dabei in der Regel subjektiv entscheiden, welche Art und/oder Stärke der Verschmutzung an den zu reinigenden Gegenständen vorliegt.

Aus dem Bereich der Geschirrspültechnik sind grundsätzlich Geschirrspülmaschinen bekannt, welche eine Erkennung der Beladung der Geschirrspülmaschine aufweisen. Beispielsweise beschreibt DE 10 2009 023 252 A1 eine Geschirrspülmaschine, welche eine Geschirrerfassungseinrichtung zur Erfassung des in den Spülbehälter eingebrachten Geschirrs aufweist. DE 100 48 081 A1 beschreibt ein Verfahren zur Erkennung der Spülgutbeladung und/oder eines Verschmutzungsgrades von Spülgut in einer programmgesteuerten Geschirrspülmaschine. DE 10 2005 014 353 A1 beschreibt eine Transportspülmaschine mit einer Spülgutsensorvorrichtung zum Detektieren von leeren Fächern einer Vielzahl von Fächern einer Transportvorrichtung. US 2002/0020435 A1 beschreibt eine Transportgeschirrspülmaschine mit einer Mehrzahl von Identifikationssensoren zur Identifikation unterschiedlicher Arten von Spülgut in der Transportgeschirrspülmaschine.

Aus der nach dem Prioritätstag der vorliegenden Anmeldung veröffentlichten EP 2 425 805 A1 ist ein Verfahren zur Reinigung von Spülgütern bekannt, insbesondere von Steckbecken, Urinflaschen oder dergleichen. Dabei wird eine programmgesteuerte Spüleinrichtung verwendet, welche eine Beladung der Spüleinrichtung mit Spülgut erkennt. In Abhängigkeit des detektierten Beladungszustands wird ein Reinigungsprogramm ausgewählt. Der Beladungszustand der Spüleinrichtung wird beispielsweise mit einer optischen Detektionseinrichtung erfasst. Als Beispiel einer optischen Detektionseinrichtung sind Infrarot-Leuchtdioden und Infrarot-Sensoren beschrieben, mittels derer ein so genanntes Lichtgitter ausgebildet wird.

In DE 10 2008 017 597 A1 wird eine Geschirrspülmaschine in Gestalt eines Programmautomaten beschrieben. Dabei wird eine Spülgutdetektorvorrichtung verwendet, mittels derer die Art des zu behandelnden Spülgutes erfasst wird. Eine Programmsteuereinrichtung ist ausgelegt, um in Abhängigkeit von der erfassten Art des zu behandelnden Spülgutes automatisch ein vorab festgelegtes oder festlegbares Behandlungsprogramm auszuwählen. Die Detektoreinrichtung kann beispielsweise eine Kamera umfassen.

In WO 2011/048575 A2 werden ebenfalls Verfahren und Vorrichtungen zum Reinigen von Gegenständen offenbart. Unter anderem wird dabei ein System beschrieben, welches mittels einer Kamera Bilder des Reinigungsguts aufnimmt, wobei eine Steuerung des Systems dementsprechend ein Programm auswählt.

In EP 1 192 893 A2 wird ein Verfahren zur Erkennung der Beladung einer programmgesteuerten Geschirrspülmaschine mit Spülgut beschrieben. Unter anderem wird während des Einbringens des Spülguts ein Verlauf von Lichtunterbrechungen in einem Lichtvorhang verzeichnet, und daraus auf die Größe und/oder die Art des Spülguts geschlossen, so dass ein folgendes Spülprogramm abgeändert oder gezielt ausgewählt werden kann.

DE 101 62 505 A1 offenbart eine Vorrichtung zum Spülen von Spülgut in einer Geschirrspülmaschine. Dabei werden wenigstens zwei Sprüheinrichtungen für bestimmte Bereiche eines Geschirrkorbs verwendet und einzeln angesteuert.

Im Bereich der Reinigungs- und Desinfektionsgeräte sind allgemein optische Sensoren, insbesondere Kameras, bislang nicht üblich. Gleichwohl ist die oben beschriebene Problematik der Erkennung der Art und Stärke der Verschmutzung bei der Reinigung von Behältern zur Aufnahme menschlicher Ausscheidungen aufgrund der unterschiedlichen Randbedingungen im Vergleich zur Geschirrspültechnik in diesem Bereich erheblich verschärft. Dies ist insbesondere dadurch bedingt, dass die Behälter, wie oben ausgeführt, größere Mengen an Abfällen aufnehmen können, beispielsweise mehrere 100 ml, welche entsorgt werden müssen. Weiterhin können, wie oben ausgeführt, infektiöse Verunreinigungen auftreten oder Verunreinigungen, welche erheblich hartnäckige Anhaftungen aufweisen. Beispielsweise muss unterschieden werden, ob ein Steckbecken lediglich mit Urin gefüllt ist, oder ob das Steckbecken zusätzlich zu den menschlichen Ausscheidungen mit Salbenresten verschmutzt ist. Letzteres kann die Wahl eines speziellen Programms bedingen, beispielsweise unter Zugabe eines Salbenreinigers. Weiterhin muss der Anwender in der Regel, abhängig von der Gefäßart, entscheiden, welches Programm gewählt wird. Beispielswese kann die Beschickung lediglich mit Urinflaschen die Wahl eines speziellen Programms bedingen.

Aus der Praxis in Krankenhäusern und Altenpflegeeinrichtungen sind üblicherweise einfach zu bedienende Tastaturen bekannt, welche typischerweise lediglich wenige Wahlmöglichkeiten bieten. Beispielsweise können Programme für leichte, mittlere oder starke Verunreinigungen ausgewählt werden. Weiterhin sind auch Ausführungen bekannt, bei denen das Programm bereits vorausgewählt ist und bei welchen ein Programmstart beispielsweise automatisch nach einem Schließen der Tür der Reinigungskammer erfolgt. Das Schließen der Tür erfolgt in der Regel manuell oder auch automatisch, beispielsweise durch Aktivierung eines berührungslosen Sensors, einer Taste oder eines Fußpedals.

Bekannte Reinigungs- und Desinfektionsgeräte weisen daher in der Praxis mehrere technische Herausforderungen oder sogar Nachteile auf. So sind insbesondere durch die manuelle, subjektiv beeinflusste Programmwahl Fehlentscheidungen möglich, was dann beispielsweise eine ungenügende Reinigung und/oder Desinfektion der Behälter zur Folge haben kann. Diese Fehlbedienung kann zu einer erhöhten Infektionsgefahr führen. Andererseits kann, um derartige Fehlbedienungen zu vermeiden, der Anwender in der Praxis versucht sein, immer das stärkste Programm auszuwählen, um in jedem Fall ein gutes Reinigungs- und Desinfektionsergebnis zu erzielen. Dies bedingt jedoch in der Praxis in vielen Fällen einen unnötig hohen Verbrauch an Ressourcen wie beispielsweise Wasser, Strom und gegebenenfalls Prozesschemikalien. Weiterhin bedingen starke Reinigungsprogramme auch längere Programmlaufzeiten, was bei anhaltend falscher Programmwahl zu Kapazitätsengpässen in der jeweiligen Betriebseinheit, beispielsweise einer Pflegestation, führen kann.

Ein weiterer Nachteil bekannter Geräte und Verfahren kann insbesondere in der Gefahr bestehen, dass Behälter in das Reinigungs- und Desinfektionsgerät eingebracht werden, für welche das Gerät nicht ausgelegt ist. Auch in diesem Fall kann eine ungenügende Reinigung und/oder Desinfektion die Folge sein. Wenn Behälter in das Reinigungs- und Desinfektionsgerät eingebracht werden, für welche dieses nicht ausgelegt ist, so können weiterhin Betriebsstörungen oder sogar Beschädigungen auftreten. So können sich beispielsweise die Behälter in dem Gerät verklemmen oder in eine Abflussöffnung einer Reinigungskammer fallen. Beides kann zu Betriebsstörungen oder auch zu Beschädigungen des Geräts führen.

### Aufgabe der Erfindung

Es ist daher eine Aufgabe der vorliegenden Erfindung, ein Reinigungs- und Desinfektionsgerät und ein Verfahren zur Behandlung mindestens eines Behälters für menschliche Ausscheidungen bereitzustellen, welche die Nachteile bekannter Reinigungsgeräte und Verfahren zumindest weitgehend vermeiden. Insbesondere sollen das Reinigungsgerät und das Verfahren flexibel auf unterschiedliche Arten von Behältern und/oder Verschmutzungen reagieren können und Fehlbedienungen zumindest weitgehend vermeiden, bei gleichzeitig qualitativ hochwertigem Reinigungsergebnis.

### Offenbarung der Erfindung

Diese Aufgabe wird gelöst durch ein Reinigungs- und Desinfektionsgerät und ein Verfahren mit den Merkmalen der unabhängigen Patentansprüche. Vorteilhafte Weiterbildungen der Erfindung, welche einzeln oder in beliebiger Kombination realisierbar sind, sind in den abhängigen Patentansprüchen dargestellt. Das beschriebene Reinigungs- und Desinfektionsgerät kann insbesondere eingerichtet sein, um ein erfindungsgemäßes Verfahren durchzuführen. Zu diesem Zweck kann das Reinigungs- und Desinfektionsgerät beispielsweise eine entsprechende Steuerung aufweisen. Umgekehrt kann das Verfahren unter Verwendung des erfindungsgemäßen Reinigungs- und Desinfektionsgeräts durchgeführt werden. Dementsprechend kann für die Beschreibung möglicher Details des Reinigungs- und Desinfektionsgeräts auf die Beschreibung optionaler Details des Verfahrens verwiesen werden und umgekehrt.

Weiterhin wird darauf hingewiesen, dass die Begriffe "aufweisen" und "umfassen" sowie entsprechende grammatikalische Abwandlungen dieser Verben im Folgenden im nichtausschließlichen Sinne verwendet werden. So beschreibt der Sachverhalt "A weist B auf" oder der Sachverhalt "A umfasst B" sowohl die Möglichkeit, dass A ausschließlich aus B besteht oder auch, alternativ, den Sachverhalt, dass A, neben B, eine oder mehrere weitere Komponenten und/oder Bestandteile beinhaltet.

In einem ersten Aspekt der vorliegenden Erfindung wird ein Reinigungs- und Desinfektionsgerät zur Behandlung mindestens eines Behälters für menschliche Ausscheidungen vorgeschlagen. Beispielsweise kann dieses Reinigungs- und Desinfektionsgerät gemäß dem oben beschriebenen Stand der Technik ausgestaltet sein, mit den nachfolgend beschriebenen zusätzlichen erfindungsgemäßen Merkmalen. Auch eine andere Ausgestaltung ist jedoch möglich. Unter einer Behandlung wird dabei allgemein eine Reinigung verstanden, welche, wie nachfolgend noch näher beschrieben wird, eine Beaufschlagung des Behälters mit mindestens einem Reinigungsfluid beinhaltet. Die Reinigung kann insbesondere derart ausgestaltet werden, dass diese den Behälter zumindest weitgehend von anhaftenden Verunreinigungen befreit. Weiterhin beinhaltet die Behandlung, zusätzlich zu einer Reinigung, auch eine Desinfektion. Unter einer Desinfektion wird allgemein im Rahmen der vorliegenden Erfindung eine Keimreduktion verstanden. Diese Keimreduktion kann beispielsweise durch eine thermische Behandlung und/oder durch eine chemische Behandlung des mindestens einen Behälters erfolgen, beispielsweise durch eine Behandlung mit einem desinfizierenden Gas und/oder mit Heißdampf. Die Desinfektion kann optional bis hin zu einer Sterilisation des Behälters führen. Unter einem Reinigungs- und Desinfektionsgerät zur Behandlung mindestens eines Behälters für menschliche Ausscheidungen ist somit im Rahmen der vorliegenden Erfindung eine Vorrichtung zu verstehen, welche eingerichtet ist, um eine Behandlung des Behälters, beinhaltend mindestens eine Reinigung und mindestens eine Desinfektion, durchzuführen. Die mindestens eine Reinigung und die mindestens eine Desinfektion können insbesondere in getrennten Programmschritten eines Programmablaufs erfolgen. Beispielsweise können das Verfahren und/oder das Reinigungs- und Desinfektionsgerät ein Programm umfassen bzw. unterstützen, in welchem mindestens ein Reinigungsschritt und mindestens ein Desinfektionsschritt als getrennte Programmschritte vorgesehen sind.

Allgemein wird unter einem Behälter für menschliche Ausscheidungen ein Behälter verstanden, welcher mindestens einen Aufnahmebereich aufweist, beispielsweise einen Hohlraum oder eine Vertiefung, in welchem eine größere Menge an menschlichen oder auch tierischen Ausscheidungen aufnehmbar ist, beispielsweise eine Menge von mindestens 50 ml, insbesondere mindestens 100 ml, vorzugsweise mindestens 200 ml oder sogar mindestens 500 ml. Zusätzlich zu dem mindestens einen Aufnahmebereich, beispielsweise dem mindestens einen Hohlraum, kann der Behälter weiterhin mindestens eine Öffnung aufweisen. Diese Öffnung kann von vorneherein vorhanden sein und/oder kann auch zu einem späteren Zeitpunkt geschaffen werden. Weiterhin kann auch während und/oder vor der Behandlung mindestens eine Öffnung geschaffen werden, beispielsweise durch ein mechanisches Öffnen des Behälters und/oder durch ein Aufschneiden und/oder Aufreißen des Behälters, beispielsweise im Rahmen des vorgeschlagenen Verfahrens. Weiterhin kann die Öffnung eingerichtet sein, um reversibel oder irreversibel geöffnet und/oder verschlossen zu werden. Der Behälter kann mindestens eine Behälterwand aufweisen, welche starr oder auch verformbar, insbesondere flexibel, ausgestaltet sein kann. So kann der Behälter beispielsweise ein Gefäß mit einer starren Behälterwand, beispielsweise aus einem oder mehreren der Materialien Glas, Kunststoff, Keramik und Metall, aufweisen. Alternativ oder zusätzlich kann der Behälter auch mindestens eine verformbare Behälterwand, beispielsweise mindestens einen Folienbeutel, insbesondere einen Folienbeutel aus einem Kunststoffmaterial, aufweisen. So kann der Behälter für menschliche Ausscheidungen beispielsweise auch ganz oder teilweise als zunächst geschlossenes Behältnis in Gestalt eines Folienbeutels ausgebildet sein.

Die Behandlung kann insbesondere, neben mindestens einer Reinigung und mindestens einer Desinfektion, auch eine Entleerung des Behälters beinhalten, beispielsweise bevor die Beaufschlagung mit dem Reinigungsfluid erfolgt. Zu diesem Zweck kann das Reinigungs- und Desinfektionsgerät beispielsweise eine oder mehrere Entsorgungsvorrichtungen aufweisen, welche eingerichtet sind, um die oben genannten Mengen an menschlichen Ausscheidungen aufzunehmen und zu entsorgen. Beispielsweise kann, wie unten noch näher ausgeführt, das Reinigungs- und Desinfektionsgerät in der Reinigungskammer einen Abfluss umfassen, beispielsweise einen Abfluss mit einem Querschnitt von mindestens 30 mm, insbesondere von mindestens 50 mm, besonders bevorzugt von mindestens 70 mm oder sogar mindestens 100 mm. Dieser Abfluss kann, wie unten noch näher ausgeführt wird, beispielsweise mit einem Siphonbogen verbunden sein. Umfasst der Behälter beispielsweise mindestens einen Folienbeutel, so kann das Reinigungs- und Desinfektionsgerät insbesondere eingerichtet sein, beispielsweise durch eine entsprechende Vorrichtung, den Folienbeutel vor und/oder während der Behandlung zu öffnen, beispielsweise aufzuschneiden und/oder aufzureißen, so dass sich dieser entleeren kann. Diese Vorrichtung kann beispielsweise fest im Reinigungs- und Desinfektionsgerät integriert sein und/oder kann durch den Anwender nach Bedarf in das Reinigungs- und Desinfektionsgerät eingesetzt werden.

Das Reinigungs- und Desinfektionsgerät kann eingerichtet sein, um gleichzeitig jeweils genau einen Behälter oder eine Mehrzahl an Behältern zu behandeln. Insbesondere kann der mindestens eine Behälter ausgewählt sein aus der Gruppe bestehend aus: einer Urinflasehe, einem Steckbecken, einer Nierenschale, einer Waschschüssel, einem Nachtgeschirr, einer Bettpfanne, einem Urinsammelbeutel, einem Sekretbeutel. Auch eine Kombination der genannten Behälter und/oder anderer Behälter ist denkbar.

Das Reinigungs- und Desinfektionsgerät umfasst mindestens eine Reinigungskammer mit mindestens einer Fluidvorrichtung zur Beaufschlagung des Behälters mit mindestens einem Reinigungsfluid. Unter einer Reinigungskammer wird dabei eine vollständig oder teilweise geschlossene Kammer verstanden, in welche der Behälter einbringbar ist und in welcher die Behandlung oder ein Teil der Behandlung des Behälters erfolgt. Insbesondere kann das Reinigungs- und Desinfektionsgerät als Einkammer-Reinigungsgerät ausgestaltet sein, wobei sämtliche Behandlungsschritte in derselben Reinigungskammer erfolgen. In der Reinigungskammer kann somit eine Beaufschlagung mit dem Reinigungsfluid erfolgen, sowie optional die Desinfektion des Behälters und/oder eine Entleerung des Behälters. Beispielsweise kann das Reinigungs- und Desinfektionsgerät eingerichtet sein, um in mindestens einem Reinigungsprogramm zunächst eine Entleerung des Behälters vorzunehmen, beispielsweise in einen Abfluss, woraufhin eine Beaufschlagung mit dem mindestens einen Reinigungsfluid erfolgt, wobei auch eine Beaufschlagung mit mehreren Reinigungsfluiden nacheinander möglich ist, woraufhin eine Desinfektion erfolgen kann, beispielsweise eine Desinfektion mit Heißdampf und/oder eine chemische Desinfektion mittels mindestens eines chemischen Desinfektionsmittels wie beispielsweise mittels mindestens eines flüssigen Desinfektionsmittels und/oder mittels mindestens eines keimabtötenden Gases wie beispielsweise Ethylenoxid. Die Reinigungskammer kann insbesondere vollständig geschlossen sein und kann beispielsweise, wie unten ausgeführt, mindestens eine Tür aufweisen.

Unter einem Reinigungsfluid ist allgemein im Rahmen der vorliegenden Erfindung eine Flüssigkeit und/oder ein Gas zu verstehen, welches eine reinigende Wirkung auf den Behälter aufweist, beispielsweise indem durch das Reinigungsfluid anhaftende Verunreinigungen von mindestens einer Oberfläche des Behälters abgespült werden. Das Reinigungsfluid kann insbesondere ein wässriges Reinigungsfluid sein, wobei Wasser verwendet werden kann, optional unter Zusatz eines oder mehrerer Reinigungsmittel und/oder Hilfsstoffe und/oder Desinfektionsmittel. Derartige Reinigungsfluide sind aus dem Stand der Technik grundsätzlich bekannt. Das Reinigungs- und Desinfektionsgerät kann beispielsweise mindestens einen Tank aufweisen, über welchen das Reinigungsfluid an die Fluidvorrichtung bereitgestellt werden kann. Alternativ oder zusätzlich kann das Reinigungs- und Desinfektionsgerät auch einen oder mehrere Anschlüsse zur Bereitstellung des Reinigungsfluids aufweisen, beispielsweise einen Frischwasseranschluss und/oder einen Heißwasseranschluss, welche beispielsweise mit einer gebäudeseitigen Versorgung verbunden werden können. Weiterhin kann, wie unten noch näher ausgeführt wird, das Reinigungs- und Desinfektionsgerät auch beispielsweise einen oder mehrere Dampferzeuger aufweisen, zur Beaufschlagung des Behälters mit Heißdampf. Weiterhin kann das Reinigungs- und Desinfektionsgerät auch einen oder mehrere Dosiertanks aufweisen, in welchen Zusatzstoffe des Reinigungsfluids gelagert werden können, beispielsweise indem diese dosiert mit anderen Komponenten des Reinigungsfluids gemischt werden, beispielsweise durch eine dosierte Einmischung in Wasser. Beispielsweise können ein oder mehrere Reinigertanks und/oder ein oder mehrere Tanks für Hilfsstoffe und/oder ein oder mehrere Tanks für Desinfektionsmittel in dem Reinigungs- und Desinfektionsgerät und/oder an dem Reinigungs- und Desinfektionsgerät umfasst sein.

Unter einer Fluidvorrichtung ist allgemein eine Vorrichtung zu verstehen, welche eingerichtet ist, um den Behälter mit dem mindestens einen Reinigungsfluid zu beaufschlagen. Beispielsweise kann eine Beaufschlagung in Form eines Besprühens und/oder eines Betropfens und/oder eines Bestrahlens des Behälters mit dem Reinigungsfluid erfolgen. Insbesondere kann die Fluidvorrichtung mindestens eine Düse aufweisen, welche mindestens einen Fluidstrahl erzeugt, der auf dem Behälter auftrifft. Beispielsweise kann die Fluidvorrichtung mindestens eine Düse umfassen, welche den Behälter aus einer oder mehreren Raumrichtungen gezielt mit einem oder mehreren Strahlen des Reinigungsfluids besprüht und/oder bestrahlt.

Das Reinigungs- und Desinfektionsgerät ist eingerichtet, um mindestens ein Reinigungsprogramm durchzuführen. Zu diesem Zweck kann das Reinigungs- und Desinfektionsgerät beispielsweise mindestens eine Steuerung umfassen. Diese Steuerung kann zentral oder auch dezentral ausgestaltet sein und/oder kann beispielsweise mindestens eine Datenverarbeitungsvorrichtung umfassen, welche programmtechnisch eingerichtet ist, um das Reinigungsprogramm zu steuern. Beispielsweise kann die Datenverarbeitungsvorrichtung eingerichtet sein, um einen, mehrere oder alle Programmparameter des Reinigungsprogramms einzustellen, beispielsweise in einer vorgegebenen Reihenfolge und/oder mit einem vorgegebenen Zeitschema.

Das Reinigungs- und Desinfektionsgerät kann also mindestens eine Steuerung aufweisen. Diese Steuerung kann insbesondere eine oder mehrere Datenverarbeitungsvorrichtungen umfassen. Die mindestens eine Steuerung kann zentral und/oder einstückig ausgestaltet sein oder kann auch dezentral ausgestaltet sein und mehrere Steuerungskomponenten umfassen. Beispielsweise kann die Steuerung einen oder mehrere Prozessoren umfassen, die optional drahtlos oder drahtgebunden miteinander verbunden sein können, um Informationen und/oder Befehle auszutauschen. Die mindestens eine Steuerung, beispielsweise die mindestens eine optionale Datenverarbeitungsvorrichtung, kann allgemein ganz oder teilweise in ein gemeinsames Modul mit der mindestens einen Reinigungskammer integriert sein, beispielsweise in ein gemeinsames Gehäuse. Alternativ oder zusätzlich kann die mindestens eine Steuerung, beispielsweise die mindestens eine Datenverarbeitungsvorrichtung, auch ganz oder teilweise außerhalb eines die Reinigungskammer umfassenden Moduls des Reinigungs- und Desinfektionsgeräts angeordnet sein, beispielsweise außerhalb eines die Reinigungsvorrichtung umschließenden Gehäuses, beispielsweise als externe Steuerung.

Die mindestens eine Steuerung kann eine oder mehrere Steuerungskomponenten umfassen, beispielsweise mehrere Datenverarbeitungsvorrichtungen, die beispielsweise über mindestens eine Schnittstelle und/oder mindestens eine Datenverbindung miteinander verbunden sein können, beispielsweise um Daten und/oder allgemeine Informationen und/oder Steuerbefehle auszutauschen. Allgemein kann die Steuerung auch mindestens eine Datenverarbeitungsvorrichtung ganz oder teilweise umfassen, welche neben einer Steuerung des Reinigungs- und Desinfektionsgeräts mindesten einem weiteren Zweck dient, beispielsweise einen PC.

Allgemein kann die optionale Steuerung also eine oder mehrere Steuerungskomponenten umfassen, welche jeweils ganz oder teilweise als Hardwarekomponenten und/oder ganz oder teilweise als Softwarekomponenten realisiert sein können.

Sind mehrere Steuerungskomponenten vorgesehen, so können diese als unterschiedliche Hardwarekomponenten ausgestaltet sein, die räumlich getrennt angeordnet sein können, beispielsweise in Form mehrerer Datenverarbeitungsvorrichtungen. Alternativ oder zusätzlich kann die Steuerung mehrere Steuerungskomponenten umfassen, welche als Softwarekomponenten in ein und derselben Hardware, beispielsweise in ein und derselben Datenverarbeitungsvorrichtung implementiert sein können.

Sind mehrere Steuerungskomponenten vorgesehen, so können diese gemeinsam an einem Ort oder auch räumlich getrennt angeordnet sein. Die Steuerungskomponenten können ohne Verbindung zueinander ausgebildet sein und/oder können paarweise oder in Gruppen drahtgebunden und/oder drahtlos miteinander verbunden sein, um unidirektional Befehle und/oder Daten auszutauschen, beispielsweise Befehle des Reinigungsprogramms und/oder Bilddaten mindestens eines mit dem Bilddetektor aufgenommenen Bildes des Behälters. Sind mehrere Steuerungskomponenten vorgesehen, so können diese auch jeweils für die Durchführung unterschiedlicher Funktionen eingerichtet sein. Beispielsweise kann mindestens eine Steuerungskomponente in Form einer Reinigungsprogrammsteuerung vorgesehen sein, wobei die Reinigungsprogrammsteuerung eingerichtet ist, um den Ablauf des Reinigungsprogramms ganz oder teilweise zu steuern. Weiterhin kann, alternativ oder zusätzlich, beispielsweise mindestens eine Steuerungskomponente in Form einer Bildverarbeitungssteuerung vorgesehen sein. Diese Bildverarbeitungssteuerung kann beispielsweise eine Datenverarbeitungsvorrichtung oder einen Teil einer Datenverarbeitungsvorrichtung umfassen, welche programmtechnisch eingerichtet ist, um ein Bild zu speichern und/oder um ein Bild zu verarbeiten.

Unter einer Bildverarbeitung kann allgemein im Rahmen der vorliegenden Erfindung ein Prozess verstanden werden, bei welchem Bilddaten mindestens einer die Bilddaten verändernden Operation unterzogen werden. Diese Operation, bei welcher es sich insbesondere um eine Rechenoperation handeln kann, kann beispielsweise mindestens eine Operation umfassen, ausgewählt aus: einer Filterung, einer Glättung, einer Datenreduktion, einer Aufhellung, einer Abdunklung, einer Kontrastveränderung. Auch andere Operationen, wie beispielsweise der Vergleich mehrerer Bilder innerhalb einer Bildfolge, sind grundsätzlich alternativ oder zusätzlich einsetzbar und dem Fachmann der Bildverarbeitung grundsätzlich bekannt.

Wenn die Steuerung mindestens eine Steuerungskomponente in Form einer Bildverarbeitungssteuerung umfasst, beispielsweise neben mindestens einer Steuerungskomponente in Form einer Reinigungsprogrammsteuerung, so können diese Steuerungskomponenten insbesondere räumlich derart getrennt ausgebildet sein, dass mindestens eine Steuerungskomponente extern ausgebildet ist, beispielsweise in einem externen Computer, insbesondere einem externen PC, welcher dann ganz oder teilweise einen Bestandteil der Steuerung bildet. Unter extern kann allgemein eine Ausgestaltung verstanden werden, bei welcher die als extern bezeichnete Komponente in einem oder mehreren Modulen ausgebildet ist, welche räumlich getrennt von einem die Reinigungskammer umfassenden Modul des Reinigungs- und Desinfektionsgeräts angeordnet sind.

Weist die Steuerung mindestens eine Steuerungskomponente in Form mindestens einer Bildverarbeitungssteuerung auf, so kann die Bildverarbeitungssteuerung ganz oder teilweise von dem Bilddetektor getrennt ausgebildet sein, beispielsweise räumlich getrennt, wobei jedoch vorzugsweise mindestens eine Datenverbindung zwischen dem Bilddetektor und der Bildverarbeitungssteuerung besteht, welche drahtgebunden und/oder drahtlos sein kann. So kann beispielsweise ein externer Computer, beispielsweise ein externer PC, zur vollständigen oder teilweisen Darstellung und/oder Abspeicherung und/oder Verarbeitung von Bilddaten eingesetzt werden. Alternativ oder zusätzlich kann die mindestens eine Bildverarbeitungssteuerung auch ganz oder teilweise in den mindestens einen Bilddetektor integriert sein und/oder räumlich mit dem Bilddetektor zusammengefasst sein. So kann beispielsweise eine Kamera mit integrierter Datenverarbeitungsvorrichtung verwendet werden, beispielsweise einem integrierten Prozessor. Der Prozessor kann programmtechnisch eingerichtet sein, um eine Datenverarbeitung und/oder eine Vorverarbeitung der Daten des Bilddetektors, beispielsweise von Bilddaten mindestens eines Bildes des Behälters, vorzunehmen, beispielsweise eine Bildverarbeitung und insbesondere eine Datenreduktion.

Unter einem Reinigungsprogramm ist allgemein eine durch einen oder mehrere Reinigungsprogrammparameter definierte zeitliche Abfolge einer Reinigung des Behälters zu verstehen. Derartige Reinigungsprogrammparameter können beispielsweise einen oder mehrere Reinigungsprogrammparameter umfassen, ausgewählt aus der Gruppe bestehend aus: mindestens einer Dauer einer Beaufschlagung des Behälters mit dem Reinigungsfluid; eine Art und/oder Zusammensetzung des Reinigungsfluids; eine Intensität einer Beaufschlagung mit dem Reinigungsfluid, beispielsweise einen Druck des Reinigungsfluids; eine Temperatur des Reinigungsfluids; eine Temperatur in der Reinigungskammer; eine Dauer eines einzelnen Reinigungsprogrammschritts; eine Abfolge unterschiedlicher Reinigungsprogrammschritte; eine Beaufschlagung des Reinigungsguts aus unterschiedlichen Düsen; eine Beaufschlagung des Reinigungsguts aus sich in der Lage ändernden Düsen, beispielsweise Hubdrehdüsen. Das Reinigungsprogramm kann insbesondere mindestens einen Reinigungsprogrammschritt aufweisen. Unter einem Reinigungsprogrammschritt wird allgemein ein definierter Abschnitt des Reinigungsprogramms verstanden, welcher sich beispielsweise durch einheitliche Reinigungsprogrammparameter auszeichnet. Beispielsweise kann das Reinigungsprogramm mehrere derartiger Reinigungsprogrammschritte umfassen. Beispielsweise kann das Reinigungsprogramm einen Entleerungsschritt umfassen und/oder einen Spülschritt, in welchem eine Beaufschlagung mit einem oder mehreren Reinigungsfluiden erfolgt, und/oder einen Desinfektionsschritt, in welchem beispielsweise eine Desinfektion mit Heißdampf und/oder anderen Desinfektionsmitteln erfolgt. Auch andere Reinigungsprogrammschritte sind grundsätzlich möglich. Beispielsweise kann das Reinigungsprogramm eine Sequenz von Reinigungsprogrammschritten aufweisen, wobei auch die Abfolge der einzelnen Reinigungsprogrammschritte und/oder die Dauer der einzelnen Reinigungsprogrammschritte und/oder die Zusammensetzung des Reinigungsprogramms als solche als Reinigungsprogrammparameter betrachtet werden können.

In dem Reinigungsprogramm wird, wie oben ausgeführt, der Behälter entleert, beispielsweise in mindestens einen Abfluss, und mit dem Reinigungsfluid beaufschlagt. Die Entleerung des Behälters kann insbesondere automatisch erfolgen. Beispielsweise kann die Entleerung des Behälters bereits bei einem Beladen des Reinigungs- und Desinfektionsgeräts mit dem Behälter erfolgen, beispielsweise indem der Behälter während des Beladens gekippt und/oder geschwenkt wird, wobei der Inhalt des Behälters beispielsweise in einen Abfluss entleert werden kann. Das Einbringen des Behälters in das Reinigungs- und Desinfektionsgerät und/oder das Verschließen des Reinigungs- und Desinfektionsgeräts kann also bereits als Teil des Reinigungsprogramms betrachtet werden. Unter einer Entleerung ist dabei allgemein ein Vorgang zu verstehen, bei welchem der Behälter, von einer Transportposition in welcher der Inhalt in dem Behälter verbleibt, in mindestens eine Entleerungsposition verbracht wird, in welcher ein möglicher Inhalt des Behälters aus dem Behälter auslaufen kann, sofern dieser Inhalt eine geeignete Konsistenz besitzt. Insbesondere kann dies unter Einfluss der eigenen Schwerkraft und/oder unter Einfluss von Zentrifugalkräften auf den Inhalt erfolgen. Beispielsweise kann der Behälter derart geschwenkt und/oder gekippt werden, dass mindestens eine Öffnung des Behälters derart nach unten weist, dass ein Auslaufen des Inhalts erfolgen kann, beispielsweise in den optionalen Abfluss. Weiterhin erfolgt in dem Reinigungsprogramm, wie oben beschrieben, eine Beaufschlagung des Behälters mit dem Reinigungsfluid, beispielsweise in einem oder mehreren Spülschritten. Dabei kann auch eine Abfolge mehrerer Spülschritte erfolgen, beispielsweise indem mehrere unterschiedliche Arten von Reinigungsfluiden sequenziell angewandt werden.

Das Reinigungs- und Desinfektionsgerät weist weiterhin mindestens einen Bilddetektor auf. Unter einem Bilddetektor ist allgemein im Rahmen der vorliegenden Erfindung eine Vorrichtung zu verstehen, welche eingerichtet ist, um mindestens ein ortsaufgelöstes optisches Signal zu erfassen. Beispielsweise kann der Bilddetektor eine Mehrzahl von in einer eindimensionalen oder zweidimensionalen Matrix angeordneten optisch sensitiven Elementen umfassen, insbesondere von optisch sensitiven Halbleiterbauelementen. Insbesondere kann der Bilddetektor mindestens eine Kamera umfassen. Insbesondere kann die mindestens eine Kamera eine CCD-Kamera umfassen. Auch andere Arten von Bilddetektoren sind alternativ oder zusätzlich einsetzbar. Insbesondere kann der Bilddetektor mindestens einen CCD-Chip und/oder mindestens einen CMOS-Chip umfassen. Weiterhin kann der Bilddetektor mindestens eine Optik umfassen, beispielsweise mindestens ein Objektiv, welche eingerichtet ist, um mindestens ein Bild auf einem sensitiven Element des Bilddetektors, beispielsweise einem Sensorchip, zu erzeugen. Der Bilddetektor kann insbesondere mindestens einen Sichtbereich aufweisen, wobei der Sichtbereich beispielsweise einen Raumwinkel und/oder eine Distanz vor dem Bilddetektor umfasst, innerhalb derer Bilddaten erfassbar sind. Beispielsweise kann der Bilddetektor mindestens eine Kamera mit festem Fokus (Fixfokus) und/oder einstellbarem Fokus und/oder mindestens eine Autofokuskamera aufweisen. Der Bilddetektor ist eingerichtet, um mindestens eine Eigenschaft des Behälters zu erkennen.

Unter einer Eigenschaft des Behälters ist dabei eine qualitativ und/oder quantitativ erfassbare Größe zu verstehen, welche den Behälter selbst und/oder in dem Behälter enthaltene Substanzen und/oder eine Position des Behälters und/oder eine Orientierung des Behälters und/oder an dem Behälter anhaftende Verunreinigungen qualitativ und/oder quantitativ charakterisiert. Um die mindestens eine Eigenschaft des Behälters zu erkennen, kann der Bilddetektor beispielsweise mindestens eine Bilderkennung aufweisen. Beispielsweise kann der Bilddetektor mindestens eine Datenverarbeitungsvorrichtung aufweisen, welche als separate Datenverarbeitungsvorrichtung eingerichtet sein kann und/oder welche auch ganz oder teilweise in eine Steuerung des Reinigungs- und Desinfektionsgeräts integriert sein kann. Beispielsweise kann diese Bilderkennung eine Mustererkennung umfassen, wobei insbesondere kommerzielle Bilderkennungssoftware zum Einsatz kommen kann. Beispielsweise kann der Bilddetektor eingerichtet sein, um mindestens ein erfasstes Bild von dem Behälter mit einer Mehrzahl von in der Datenverarbeitungsvorrichtung und/oder einem Speicher der Datenverarbeitungsvorrichtung hinterlegten Mustern und/oder Bildern zu vergleichen, wie unten exemplarisch noch näher ausgeführt wird.

Das Datenverarbeitungsgerät ist weiterhin eingerichtet, um das Reinigungsprogramm entsprechend der erkannten Eigenschaft zu beeinflussen. Beispielsweise kann das Reinigungs- und Desinfektionsgerät, wie oben ausgeführt, zu diesem Zweck eine zentrale und/oder eine dezentrale Steuerung umfassen, beispielsweise eine Steuerung mit mindestens einer Datenverarbeitungsvorrichtung, welche programmtechnisch eingerichtet ist. Unter einer Beeinflussung des Reinigungsprogramms ist allgemein dabei ein Vorgang zu verstehen, bei welchem mindestens ein Reinigungsprogrammparameter des Reinigungsprogramms verändert wird. Beispielsweise kann es sich dabei um einen oder mehrere der oben genannten Reinigungsprogrammparameter handeln. Beispiele von Beeinflussungen werden unten noch näher erläutert. Die Beeinflussung kann automatisch erfolgen, beispielsweise indem das Reinigungsprogramm automatisch einen oder mehrere Reinigungsprogrammparameter beeinflusst. Alternativ oder zusätzlich kann die Beeinflussung auch unter Mitwirkung eines Anwenders oder Anwenders des Reinigungs- und Desinfektionsgeräts erfolgen, beispielsweise indem mindestens ein Hinweis und/oder mindestens ein Vorschlag und/oder mindestens eine Warnung an einen Anwender ausgegeben wird. Auch dies kann als Beeinflussung des Reinigungsprogramms gesehen werden, da eine derartige Ausgabe von Informationen an einen Anwender grundsätzlich Bestandteil des Reinigungsprogramms sein kann. Die Beeinflussung des Reinigungsprogramms kann insbesondere eine Zusammensetzung des Reinigungsprogramms aus einem oder mehreren Reinigungsprogrammschritten oder einer Ausgestaltung eines oder mehrerer Reinigungsprogrammschritte umfassen.

Wie oben ausgeführt, kann das Reinigungs- und Desinfektionsgerät insbesondere eine oder mehrere Steuerungen aufweisen. Diese mindestens eine Steuerung kann beispielsweise mindestens eine Datenverarbeitungsvorrichtung umfassen, beispielsweise mindestens einen Mikrocomputer und/oder Mikrocontroller. Weiterhin kann die Steuerung beispielsweise eine oder mehrere Schnittstellen umfassen, zum Austausch von Informationen und/oder Befehlen mit mindestens einem Anwender und/oder Benutzer und/oder mit mindestens einem weiteren Gerät. Beispielsweise kann diese mindestens eine Schnittstelle mindestens ein Display umfassen, auf welchem Informationen an einen Anwender bereitgestellt werden können und/oder eine oder mehrere akustische und/oder optische und/oder haptische Warnvorrichtungen. Weiterhin kann die mindestens eine Schnittstelle mindestens eine Tastatur und/oder eine andere Art von Mensch-Maschine-Schnittstelle umfassen. Weiterhin kann die Schnittstelle mindestens eine Computerschnittstelle umfassen, zur Anbindung des Reinigungs- und Desinfektionsgeräts an einen anderen Computer und/oder ein Datennetzwerk.

In einer bevorzugten Ausgestaltung des Reinigungs- und Desinfektionsgeräts kann der Bilddetektor insbesondere außerhalb der Reinigungskammer angeordnet sein. Beispielsweise kann der Bilddetektor auf einer Außenseite einer Hülle der Reinigungskammer angeordnet sein, beispielsweise in einem Deckelbereich und/oder in einem Frontbereich des Reinigungs- und Desinfektionsgeräts. Der Bilddetektor kann insbesondere eingerichtet sein, um die Eigenschaft vor und/oder während eines Einbringens des Behälters in die Reinigungskammer zu erkennen. Beispielsweise kann der Bilddetektor auf einer Außenseite der Reinigungskammer angeordnet sein, wobei eine Eingabeöffnung der Reinigungskammer, beispielsweise eine Türöffnung einer Tür der Reinigungskammer, in einem Sichtbereich des Bilddetektors angeordnet ist, also beispielsweise innerhalb eines Raumwinkelbereichs, innerhalb dessen der Bilddetektor Bilddaten erfassen kann. Beispielsweise kann die Eingabeöffnung eine bewegbare Tür aufweisen, insbesondere eine Klappe. Beispielsweise kann die Eingabeöffnung eine Klappe mit einem Scharnier aufweisen, welche in einem Frontbereich und/oder einem Deckelbereich der Reinigungskammer angeordnet ist. Beispielsweise kann die Klappe nach vorne, zu einem Anwender und/oder Benutzer hin, aufschwenkbar sein. Insbesondere kann mindestens eine Halterung zur Aufnahme des Behälters mit der Tür verbunden sein. Beispielsweise kann diese Halterung dergestalt sein, dass bei geöffneter Tür der Behälter derart orientiert ist, dass der Inhalt des Behälters im Behälter verbleibt, beispielsweise indem eine Öffnung des Behälters nach oben orientiert ist. Während des Schließens der Tür kann der Behälter dann beispielsweise geschwenkt werden, sodass beispielsweise während und/oder nach dem Schließen der Inhalt aus dem Behälter auslaufen kann. Beispielsweise kann dabei die Öffnung des Behälters in eine horizontale Richtung und/oder in eine nach unten weisende Richtung geschwenkt werden.

Wie oben ausgeführt, kann der Bilddetektor, insbesondere die Kamera, vorzugsweise außerhalb der Reinigungskammer angeordnet sein. Der Bilddetektor kann eingerichtet sein, um eine Bilderfassung des Behälters vor und/oder während des Einbringens des Behälters in die Reinigungskammer durchzuführen. Dabei kann mindestens ein Bild des Behälters vor und/oder während des Einbringens des Behälters in die Reinigungskammer erfasst und vorzugsweise abgespeichert werden. Unter einem Bild ist allgemein eine eindimensionale, zweidimensionale oder dreidimensionale Menge an optischen Informationen zu verstehen, wobei vorzugsweise ein zweidimensionales Bild aufgenommen wird.

Ein Vorteil der Anordnung des Bilddetektors außerhalb der Reinigungskammer kann insbesondere in einer Verbesserung der Qualität der Bilderfassung liegen. So kann in der Regel ein außerhalb der Reinigungskammer angeordneter Bilddetektor, insbesondere eine außerhalb der Reinigungskammer angeordnete Kamera, wesentlich bessere Ergebnisse liefern als bei einer Anordnung innerhalb der Reinigungskammer. Dies kann beispielsweise dadurch bedingt sein, dass in der Regel die Gefahr einer Verschmutzung einer Optik des Bilddetektors, insbesondere einer Verschmutzung eines Objektivs, beispielsweise einer Kamera, durch die Anordnung außerhalb der Reinigungskammer deutlich verringert wird. Zudem kann eine Ausschüttung des Inhalts des Behälters zu einer verfälschen Information über die Art einer Beladung des Behälters führen und somit beispielsweise zu einer Verfälschung der zu erkennenden Eigenschaft.

Ein weiterer Vorteil der Anordnung des Bilddetektors außerhalb der Reinigungskammer liegt darin, dass bereits vor einer Einbringung des Behälters in die Reinigungskammer entsprechende Maßnahmen ergriffen werden können, die sich aus der von dem Bilddetektor erfassten Eigenschaft ergeben können. So kann beispielsweise Bedienpersonal bereits vor, während oder bei der Beladung erforderliche Korrekturen vornehmen, beispielsweise ohne gesundheitliche Risiken oder hygienische Risiken, im Gegensatz zu bekannten Anordnungen. Auch die Gefahr einer Betriebsstörung kann deutlich reduziert werden im Vergleich zu Vorrichtungen, bei welchen eine Detektion erst innerhalb der Reinigungskammer erfolgt. So besteht grundsätzlich eine deutlich verringerte Gefahr dahingehend, dass Betriebsstörungen durch fehlerhafte, ungeeignete oder in anderer Weise falsch eingebrachte Behälter entstehen, da beispielsweise schon vor einem Schließen einer Tür der Reinigungskammer und/oder unmittelbar zu Beginn des Schließens einer Tür der Reinigungskammer eine Erkennung von Fehlern durchgeführt werden kann.

Unter einer Halterung ist im Rahmen der vorliegenden Erfindung allgemein eine Vorrichtung zu verstehen, welche eingerichtet ist, um den Behälter aufzunehmen und relativ zur Halterung zu fixieren. Beispielsweise kann diese Halterung mindestens eine Schiene umfassen, in welche der Behälter eingeschoben werden kann. Alternativ oder zusätzlich kann die Halterung auch einen oder mehrere Bügel und/oder einen oder mehreren Hohlräume umfassen, in welche der Behälter eingeschoben und/oder eingebracht werden kann. Dabei kann das Reinigungs- und Desinfektionsgerät eine feste Halterung aufweisen oder auch eine austauschbare Halterung, beispielsweise indem eine Art der Halterung auf den Typ des Behälters angepasst werden kann.

Der Bilddetektor kann insbesondere eingerichtet sein, um die Eigenschaft des Behälters in der Halterung bei geöffneter Tür zu erkennen. Beispielsweise kann dies dadurch erfolgen, dass der Bilddetektor außerhalb der Reinigungskammer angeordnet ist, beispielsweise auf einer Außenseite der Reinigungskammer, wobei der Bilddetektor einen Eingabebereich, beispielsweise einen Schwenkbereich der Tür, beobachtet. Auf diese Weise kann die mindestens eine Eigenschaft beispielsweise bereits vor dem Einbringen des Behälters in die Reinigungskammer, insbesondere vor einem Schließen der Tür, erkannt werden. Diese Ausgestaltung bietet insbesondere, wie oben bereits ausgeführt und wie unten noch näher ausgeführt wird, den Vorteil, dass Fehlbeladungen und/oder Fehlbedienungen zumindest teilweise bereits vor einem Schließen des Behälters erkannt werden können.

Das Reinigungs- und Desinfektionsgerät, insbesondere der Bilddetektor, kann insbesondere eingerichtet sein, um vor dem Schließen der Tür und/oder während des Schließens des der Tür mindestens ein Bild des mindestens einen in die Reinigungskammer einzubringenden Behälters aufzunehmen. Insbesondere kann unmittelbar vor dem Schließen der Tür mindestens ein Bild des Behälters aufgenommen werden, beispielsweise innerhalb eines Zeitraums von 0-5 s vor dem Schließen der Tür. Das mindestens eine Bild kann insbesondere in einem Datenspeicher abgespeichert werden.

Alternativ oder zusätzlich kann das Reinigungs- und Desinfektionsgerät, insbesondere der Bilddetektor, insbesondere auch eingerichtet sein, um nach einem erneuten Öffnen der Tür und/oder während eines erneuten Öffnens der Tür, insbesondere nach Durchführung eines Reinigungsprogramms, mindestens ein Bild des Behälters aufzunehmen. Insbesondere kann unmittelbar nach dem erneuten Öffnen der Tür mindestens ein Bild des nunmehr vorzugsweise gereinigten Behälters aufgenommen werden, beispielsweise innerhalb eines Zeitraums von 0-5 s nach dem Öffnen der Tür. Das mindestens eine Bild kann wiederum insbesondere in einem Datenspeicher abgespeichert werden.

Ist das Reinigungs- und Desinfektionsgerät eingerichtet, um mindestens ein Bild des Behälters aufzunehmen und abzuspeichern, beispielsweise vor und/oder nach Durchführung eines Reinigungsprogramms, so kann dieses Bild optional mit einer oder mehreren Zusatzinformationen abgespeichert werden. Beispielsweise kann das mindestens eine Bild mit mindestens einem Zeitstempel und/oder mit mindestens einer Zuordnung zu einem Reinigungsprogramm, beispielsweise einem abgelaufenen Reinigungsprogramm, welchem der Behälter unterzogen wird oder unterzogen wurde, abgespeichert werden.

Alternativ oder zusätzlich kann die mindestens eine Zusatzinformation mindestens eine Information über einen Benutzer enthalten, beispielsweise eine bestimmte Person des Bedienpersonals. Diese Zuordnung kann beispielsweise anhand einer Identifikationsnummer erfolgen. Beispielsweise kann das Reinigungs- und Desinfektionsgerät eingerichtet sein, um einen elektronischen Identifikator eines Benutzers zu erfassen, beispielsweise mittels eines RFID-Chips. Die Identifikation des Benutzers kann als Zusatzinformation oder als Teil der Zusatzinformation gemeinsam mit dem mindestens einen Bild abgespeichert werden. Auf diese Weise kann beispielsweise eine Zuordnung bestimmter Vorgänge zu einem bestimmten Benutzer erfolgen. Der Betreiber des Reinigungs- und Desinfektionsgeräts hat somit allgemein vorzugsweise die Möglichkeit, anhand des mindestens einen Bildes eine korrekte Bedienung zu überwachen und gegebenenfalls Fehlbedienungen zu erkennen. Gegebenenfalls können dann Schulungsmaßnahmen eingeleitet werden.

Das Reinigungs- und Desinfektionsgerät kann insbesondere eingerichtet sein, um anhand des mindestens einen vor und/oder nach Ablauf des Reinigungsprogramms aufgenommenen Bildes des Behälters, gegebenenfalls unter Einbeziehung einer oder mehrerer Zusatzinformationen, eine Dokumentation des Reinigungsvorgangs bereitzustellen. Wird mindestens ein Bild aufgenommen und abgespeichert, so ist insbesondere eine Echtzeit-Bildverarbeitung in der Regel nicht erforderlich und kann vorzugsweise entfallen. Gleichzeitig kann ein Betreiber des Reinigungs- und Desinfektionsgeräts anhand der Bilder nachweisen, dass das Bedienpersonal das Reinigungs- und Desinfektionsgerät korrekt bedient und geeignete Programme verwendet.

Das Reinigungs- und Desinfektionsgerät, insbesondere der Bilddetektor, kann insbesondere eingerichtet sein, um mindestens ein mittels des Bilddetektors aufgenommenes Bild, beispielsweise mindestens ein Bild des Behälters, abzuspeichern und/oder weiterzuleiten. Beispielsweise kann das Reinigungs- und Desinfektionsgerät, insbesondere der Bilddetektor, eingerichtet sein, um das mindestens eine Bild über mindestens eine Schnittstelle und/oder über mindestens eine Datenverbindung drahtlos und/oder drahtgebunden an mindestens eine weitere Vorrichtung weiterzuleiten. So kann das Reinigungs- und Desinfektionsgerät, insbesondere der Bilddetektor, eingerichtet sein, um das mindestens eine Bild nach außen zu übergeben und/oder zu verschicken, so dass dieses Bild beispielsweise an einer Datenverarbeitungsvorrichtung außerhalb des Reinigungs- und Desinfektionsgeräts zur Verfügung steht. Beispielsweise kann das mindestens eine Bild in einem Computer, beispielsweise einem PC, in einer Hygiene-Leitwarte gespeichert und optional verarbeitet werden. Das mindestens eine Bild kann also optional innerhalb des Reinigungs- und Desinfektionsgeräts und/oder in einem externen Computer abgespeichert, dargestellt und/oder verarbeitet werden.

Wird mindestens ein Bild an eine externe Datenverarbeitungsvorrichtung übermittelt, so kann diese externe Datenverarbeitungsvorrichtung dann beispielsweise eingerichtet sein, um entsprechend einem Ergebnis einer Bildverarbeitung das mindestens eine Reinigungs- und Desinfektionsgerät zu beeinflussen, beispielsweise das mindestens eine Reinigungsprogramm zu beeinflussen. In diesem Sinne kann die externe Datenverarbeitungsvorrichtung dann begrifflich Teil des Reinigungs- und Desinfektionsgeräts sein, beispielsweise als Teil einer Steuerung des Reinigungs- und Desinfektionsgerät, auch wenn diese externe Datenverarbeitungsvorrichtung beispielsweise außerhalb eines die Reinigungskammer umschließenden Gehäuses des Reinigungs- und Desinfektionsgeräts angeordnet ist. Alternativ oder zusätzlich kann die externe Datenverarbeitungsvorrichtung auch gemeinsam mit dem Reinigungs- und Desinfektionsgerät ein Reinigungs- und Desinfektionssystem bilden.

In der externen Datenverarbeitungsvorrichtung, beispielsweise dem externen PC, können dann beispielsweise die gleichen Routinen ablaufen, wie oben oder nachfolgend bezüglich des Reinigungs- und Desinfektionsgeräts selbst beschrieben. Beispielsweise können das Reinigungs- und Desinfektionsgerät und/oder die externe Datenverarbeitungsvorrichtung eingerichtet sein, um einen Vergleich durchzuführen, ob eine Beladung des Reinigungs- und Desinfektionsgeräts zulässig war, ob der Behälter richtig eingesetzt war oder ähnliche Auswertungsoperationen.

Das Reinigungs- und Desinfektionsgerät kann allgemein insbesondere eingerichtet sein, um mindestens eine Warnung an einen Benutzer auszugeben, beispielsweise eine visuelle Warnung und/oder eine akustische Warnung. Die Warnung kann beispielsweise entsprechend einem Ergebnis einer Verarbeitung des mindestens einen Bildes ausgegeben werden. Wenn eine Verarbeitung des mindestens einen Bildes, beispielsweise durch die Steuerung und/oder durch eine externe Datenverarbeitungsvorrichtung, sehr zeitnah zu einem Programmstart des Reinigungsprogramms erfolgt (beispielsweise innerhalb einer Zeitspanne von nicht mehr als einer Minute, insbesondere von nicht mehr als 30 Sekunden) und wenn dabei Auffälligkeiten erkannt werden, so können die Steuerung und/oder die externe Datenverarbeitungsvorrichtung eingerichtet sein, um vor und/oder noch während des Programmablaufs eine Rückmeldung an das Reinigungs- und Desinfektionsgerät zu erzeugen. So kann das Reinigungs- und Desinfektionsgerät beispielsweise eingerichtet sein, um einem Benutzer an einem Programmende eine Warnung auszugeben, dass das Reinigungsergebnis besonders zu begutachten ist.

Das Reinigungs- und Desinfektionsgerät und/oder eine externe Datenverarbeitungseinrichtung können optional eingerichtet sein, um eine oder mehrere Informationen entsprechend einem Ergebnis einer Verarbeitung des mindestens einen Bildes an einen Benutzer und/oder eine verantwortliche Person zu kommunizieren, beispielsweise elektronisch, insbesondere über Email und/oder SMS. So können das Reinigungs- und Desinfektionsgerät und/oder eine externe Datenverarbeitungseinrichtung eingerichtet sein, um, vorzugsweise unmittelbar, bei Auffälligkeiten oder gesammelt in wählbaren Zeitabständen eine Mail beispielsweise an den Hygienebeauftragten zu versenden. Der Hygienebeauftragte kann dann beispielsweise entsprechende Schulungsmaßnahmen einleiten.

Ein Vorteil einer Verwendung mindestens einer externen Datenverarbeitungsvorrichtung, als Teil einer Steuerung des Reinigungs- und Desinfektionsgeräts und/oder als externe Vorrichtung, kann insbesondere in einer Bereitstellung von Ressourcen liegen. So lassen sich Ressourcen, beispielsweise Rechenkapazität und/oder Speicherplatz, kostengünstig bereitstellen, auch bei beengten Platzverhältnissen im Reinigungs- und Desinfektionsgerät. Zudem können Ressourcen von mehreren Reinigungs- und Desinfektionsgeräten gemeinsam genutzt werden.

Die Steuerung kann insbesondere mindestens eine Hygienesteuerungskomponente umfassen. Die Hygienesteuerungskomponente kann insbesondere eine Steuerungskomponente sein, welche eingerichtet ist, um einen Benutzer bei einer Bewertung einer Reinigungswirkung und/oder Hygienisierung des Behälters durch das Reinigungsprogramm zu unterstützen. So kann die Hygienesteuerungskomponente insbesondere eine oder mehrere der folgenden Funktionen aufweisen: eine Darstellung mindestens eines Bildes des Behälters auf einem Bildschirm, eine Dokumentation von Bilddaten, eine Speicherung von Bilddaten, eine Datenbankfunktion, eine Weiterleitungsfunktion von Bilddaten, beispielsweise über Email und/oder MMS. Die Hygienesteuerungskomponente kann insbesondere ganz oder teilweise auf mindestens einer externen Datenverarbeitungsvorrichtung implementiert sein, insbesondere einem externen PC. Insbesondere kann die Hygienesteuerungskomponente ganz oder teilweise als Softwarekomponente der Steuerung implementiert sein. So kann die Hygienesteuerungskomponente beispielsweise einen Hygieneassistenten in Form einer Softwarekomponente, beispielsweise ein kleines EDV-Programm, umfassen, welche insbesondere ganz oder teilweise auf einer externen Datenverarbeitungsvorrichtung und/oder auf einer zentralen Datenverarbeitungsvorrichtung installiert sein kann.

Ist mindestens eine Hygienesteuerungskomponente vorgesehen, so kann ein Betreiber des Reinigungs- und Desinfektionsgeräts insbesondere in der Hygienesteuerungskomponente, beispielsweise im Hygieneassistenten, einen Stichprobenmodus definieren. Die Hygienesteuerungskomponente kann insbesondere eingerichtet sein, um Anfragen zur Bewertung eines Betriebsablaufs zu versenden. Dies kann beispielsweise in Form einer Email und/oder MMS mit Bildern an einen Verantwortlichen erfolgen, beispielsweise einen Hygienebeauftragten. Der Verantwortliche kann die Bilder ansehen und bewerten und kann entscheiden, ob der Prozessablauf beanstandungsfrei ist oder ob sich ein Handlungsbedarf ergibt. Eine Bildverarbeitung und/oder Bildbewertung können dann auch ganz oder teilweise, alternativ oder zusätzlich zu einer Bildverarbeitung durch die Steuerung, durch einen Menschen erfolgen. Das Ergebnis der menschlichen Beurteilung kann beispielsweise wiederum an die Steuerung kommuniziert werden, beispielsweise mittels Schaltflächen und/oder einer Antwortmail an den Hygieneassistenten. Für den Betreiber des Reinigungs- und Desinfektionsgeräts kann sich schon mit dieser einfachen Methode eine verbesserte Dokumentation der Arbeitsabläufe ergeben.

Das Reinigungs- und Desinfektionsgerät kann weiterhin mindestens eine Beleuchtungsvorrichtung aufweisen. Diese Beleuchtungsvorrichtung kann beispielsweise eingerichtet sein, um einen Sichtbereich des Bilddetektors zumindest teilweise zu beleuchten. Dementsprechend kann optional diese Beleuchtungsvorrichtung ebenfalls außerhalb der Reinigungskammer angeordnet sein, beispielsweise auf einer Außenseite der Reinigungskammer. Die Beleuchtungsvorrichtung kann beispielsweise mindestens eine Glühlampe und/oder mindestens eine Gasentladungslampe und/oder mindestens eine Halbleiterlichtquelle umfassen, beispielsweise mindestens eine Leuchtdiode. Die Beleuchtungsvorrichtung kann zur Emission von sichtbarem Licht eingerichtet sein. Alternativ oder zusätzlich kann die Beleuchtungsvorrichtung jedoch auch elektromagnetische Strahlung in einem anderen Spektralbereich emittieren, beispielsweise in einem infraroten und/oder ultravioletten Spektralbereich. Auf diese Weise können beispielsweise besondere Arten von an dem Behälter anhaftenden Verschmutzungen und/oder besondere Arten von Inhalt des Behälters erkannt werden.

Das Reinigungs- und Desinfektionsgerät kann, wie oben beschrieben, insbesondere mindestens eine Halterung zur Aufnahme des Behälters aufweisen. Diese Halterung kann allgemein beispielsweise mindestens eine Beladungsposition außerhalb der Reinigungskammer aufweisen, wobei das Reinigungsgut durch einen Anwender in der Beladungsposition in die Halterung einbringbar und/oder aus der Halterung entnehmbar ist. Im Folgenden werden die Begriffe des Benutzers und des Anwenders dabei synonym verwendet, unabhängig davon, ob es sich hierbei beispielsweise um geschultes Personal handelt oder nicht. Die Halterung kann weiterhin mindestens eine Reinigungsposition innerhalb der Reinigungskammer aufweisen, wobei der Behälter in der Reinigungsposition in der Reinigungskammer angeordnet ist und mit dem Reinigungsfluid beaufschlagbar ist.

Die Beladungsposition kann insbesondere die oben beschriebene Position bei geöffneter Tür des Reinigungs- und Desinfektionsgeräts sein, wobei die Halterung beispielsweise mit der Tür verbunden ist. Allgemein kann der Bilddetektor insbesondere eingerichtet sein, um die Eigenschaft der Beladungsposition zu erkennen, beispielsweise bei geöffneter Tür.

Wie oben ausgeführt, kann das Reinigungs- und Desinfektionsgerät insbesondere eingerichtet sein, um den Behälter in der Reinigungsposition und/oder bei einem Übergang von der Beladungsposition in die Reinigungsposition zu entleeren, insbesondere in einen Abfluss. Die Halterung kann insbesondere, wie oben ausgeführt, in einer Tür der Reinigungsvorrichtung angeordnet sein, insbesondere in einer frontseitigen Klappe. Der Behälter kann insbesondere bei einem Übergang von der Beladungsposition in die Reinigungsposition geschwenkt werden, wobei der Behälter um eine oder mehrere virtuelle oder reale Achsen gedreht wird.

Weitere mögliche Ausgestaltungen der Erfindung betreffen die Eigenschaft, welche mittels des Bilddetektors erkannt werden kann. Wie oben beschrieben, kann die mindestens eine erkennbare Eigenschaft den Behälter selbst und/oder einen Inhalt des Behälters und/oder an dem Behälter anhaftende Verunreinigungen und/oder die Position und/oder Lage des Behälters in der Halterung betreffen. Insbesondere kann die Eigenschaft ausgewählt sein aus der Gruppe bestehend aus: einer Art des Behälters, insbesondere einem Behältertyp und/oder einer Behälterform; einer Befüllung des Behälters mit menschlichen Ausscheidungen, insbesondere eine Füllmenge und/oder eine Art der Befüllung und vorzugsweise einer Erkennung, ob eine wässrige Befüllung vorliegt oder nicht; einem Verschmutzungsgrad des Behälters; einer Art einer Verschmutzung des Behälters, beispielsweise einer Verschmutzung mit Salbenresten, einer Eignung des Behälters zur Reinigung in dem Reinigungs- und Desinfektionsgerät.

Unter einem Verschmutzungsgrad kann dabei im Rahmen der vorliegenden Erfindung allgemein eine Einstufung einer Verschmutzung mindestens einer Oberfläche des Behälters verstanden werden, bei welcher mindestens eine von mindestens zwei Kategorien von Verschmutzungen ausgewählt wird. Beispielsweise kann der Verschmutzungsgrad durch zwei, drei oder mehr Kategorien vorgegeben sein, beispielsweise indem die Kategorien "sauber" und "verschmutzt" verwendet werden oder die Kategorien "sauber", "leicht verschmutzt" und "stark verschmutzt" oder eine größere Anzahl an Kategorien. Auch eine stufenlose Beschreibung des Verschmutzungsgrades ist grundsätzlich denkbar, beispielsweise indem über einen Oberflächenbereich des Behälters hinweg eine Reflektivität der Oberfläche gemittelt erfasst wird, wobei ein Rückgang der Reflektivität beispielsweise auf eine höhere Verschmutzung schließen lassen kann. Verschiedene andere Ausgestaltungen der Beurteilung des Verschmutzungsgrades sind denkbar. Der Verschmutzungsgrad kann beispielsweise optisch erfasst werden, beispielsweise mittels einer Reflexionsmessung und/oder einer Auswertung eines Kamerabilds, oder auf andere Weise.

Weitere mögliche Ausgestaltungen der Erfindung betreffen die Art der Beeinflussung des Reinigungsprogramms entsprechend der erkannten Eigenschaft. Wie oben ausgeführt, kann dabei insbesondere mindestens ein Reinigungsprogrammparameter beeinflusst werden. Insbesondere kann die Beeinflussung des Reinigungsprogramms ausgewählt sein aus der Gruppe bestehend aus: einer Auswahl eines geeigneten Reinigungsprogramms aus einer Liste von Reinigungsprogrammen; ein Vorschlag eines geeigneten Reinigungsprogramms an einen Benutzer des Reinigungs- und Desinfektionsgeräts; einer Beeinflussung mindestens eines Parameters des Reinigungsprogramms, insbesondere eines Parameters ausgewählt aus der Gruppe bestehend aus mindestens eines Reinigungsprogrammschritts des Reinigungsprogramms, einer Änderung mindestens einer Konzentration mindestens einer Komponente des Reinigungsfluids, insbesondere einer Konzentration mindestens eines Reinigungsmittels und/oder mindestens eines Desinfektionsmittels in dem Reinigungsfluid und einer Temperatur des Reinigungsfluids; einer Ansteuerung von verschiedenen Düsen; einer Beaufschlagung des Reinigungsguts aus sich in der Lage ändernden Düsen; einer Einfügung mindestens eines zusätzlichen Reinigungsprogrammschritts zu dem Reinigungsprogramm; einer Verhinderung der Durchführung des Reinigungsprogramms und/oder mindestens eines folgenden Reinigungsprogrammschritts des Reinigungsprogramms, beispielsweise bei Erkennung eines ungeeigneten Behälters und/oder einer ungeeigneten Befüllung des Behälters und/oder einer falschen Position des Behälters; eine Ausgabe mindestens einer Warnung und/oder mindestens eines Hinweises an einen Benutzer.

Das Reinigungs- und Desinfektionsgerät kann insbesondere, wie oben beschrieben, mindestens einen Abfluss aufweisen. Beispielsweise kann der mindestens eine Abfluss in einem Bodenbereich der Reinigungskammer angeordnet sein. Beispielsweise kann die Reinigungskammer im Bodenbereich zumindest teilweise trichterförmig und/oder geneigt auf den Abfluss zulaufen. Insbesondere kann der Abfluss mindestens einen Geruchsverschluss aufweisen, also eine Vorrichtung, welche eingerichtet ist, um Gase aus mindestens einem mit dem Abfluss verbundenen Abflussrohr vom Inneren der Reinigungskammer fernzuhalten. Beispielsweise kann der Geruchsverschluss mindestens einen Siphonbogen aufweisen. Das Reinigungs- und Desinfektionsgerät kann insbesondere eingerichtet sein, um den Behälter in dem Reinigungsprogramm in den Abfluss hinein zu entleeren. Wie oben ausgeführt, kann diese Entleerung in dem Reinigungsprogramm eine automatische Entleerung umfassen und/oder eine Entleerung, welche beispielsweise während eines Einbringens des Behälters in die Reinigungskammer erfolgt, beispielsweise während eines Schließens einer Tür, wobei beispielsweise eine Halterung für den Behälter mit der Tür verbunden ist und während des Schließens der Tür geschwenkt wird. Das Schließen der Tür kann dabei ganz oder teilweise durch einen Antrieb angetrieben werden, beispielsweise durch einen Motor, eine Mechanik, eine Hydraulik oder eine Pneumatik und/oder kann auch ganz oder teilweise durch Muskelkraft angetrieben werden.

Wie beispielsweise aus DE 103 48 344 A1 bekannt, kann das Reinigungs- und Desinfektionsgerät weiterhin mindestens einen Bypass aufweisen, wobei über den Bypass Gas und Dampf aus der Reinigungskammer unter Umgehung des Geruchsverschlusses, beispielsweise unter Umgehung des Siphonbogens, in den Abfluss ableitbar sind, vorzugsweise unter Druck. Beispielsweise kann der Bypass eine oder mehrere Rohrleitungen umfassen, welche die Reinigungskammer mit dem Abfluss unter Umgehung des Geruchsverschlusses verbinden. Beispielsweise kann der Bypass weiterhin mindestens ein Rückschlagventil und/oder mindestens eine andere Art von Ventil aufweisen, welche ein Eindringen von Gasen aus dem Abfluss und/oder einem mit dem Abfluss verbundenen Abflussrohr durch den Bypass in die Reinigungskammer ganz oder teilweise verhindern.

Wie oben beschrieben, kann die Fluidvorrichtung insbesondere mindestens einen Flüssigkeitstank sowie mindestens eine Düse zur Beaufschlagung des Behälters mit einer Flüssigkeit umfassen. Weiterhin kann die Fluidvorrichtung insbesondere mindestens einen Dampferzeuger zur Erzeugung von Heißdampf und zur Beaufschlagung des Behälters mit dem Heißdampf aufweisen. Für mögliche Ausgestaltungen kann wiederum beispielsweise auf die DE 103 48 344 A1 verwiesen werden.

Eine weitere mögliche Ausgestaltung des Reinigungs- und Desinfektionsgeräts betrifft die Möglichkeit, das Reinigungs- und Desinfektionsgerät für bestimmte Arten von Behältern und/oder Inhalte und/oder Verunreinigungen anzupassen. Insbesondere kann das Reinigungs- und Desinfektionsgerät eingerichtet sein, um mindestens ein Lernprogramm durchzuführen. Beispielsweise kann die mindestens eine Steuerung des Reinigungs- und Desinfektionsgeräts programmtechnisch eingerichtet sein, um dieses Lernprogramm zu ermöglichen. Beispielsweise kann in dem Lernprogramm mindestens ein Behälter mit mindestens einer bekannten Eigenschaft durch einen Benutzer in einen Sichtbereich des Bilddetektors eingebracht werden, insbesondere in einer Halterung. Mittels des Bilddetektors kann die Eigenschaft erkannt werden, und durch den Benutzer kann eine der Eigenschaft entsprechende Beeinflussung des Reinigungsprogramms vorgegeben und in dem Reinigungs- und Desinfektionsgerät abgespeichert werden. Auf diese Weise können, durch Vorgabe des Benutzers, eine oder mehrere Zuordnungen entstehen, bei welchen einer oder mehreren Eigenschaften jeweils eine oder mehrere Beeinflussungen des Reinigungsprogramms zugeordnet werden, welche später, nach Durchführung des Lernprogramms, im Reinigungsbetrieb verwendbar sind. Beispielsweise kann ein Benutzer auf diese Weise für verschiedene Behältertypen und/oder Behälterarten jeweils bestimmte Reinigungsprogramme vorgeben.

Insbesondere kann das Reinigungs- und Desinfektionsgerät, beispielsweise eine Steuerung des Reinigungs- und Desinfektionsgeräts, mindestens eine Datenbank aufweisen. In der Datenbank kann eine Mehrzahl erkennbarer Eigenschaften sowie zu jeder Eigenschaft mindestens eine durchzuführende Beeinflussung des Reinigungsprogramms hinterlegt sein. Diese Datenbank kann beispielsweise durch wiederholte Durchführung des oben beschriebenen Lernprogramms ergänzt werden. Das Reinigungs- und Desinfektionsgerät kann allgemein beispielsweise derart eingerichtet sein, dass die Durchführung des Lernprogramms auf einen bestimmten Benutzerkreis eingeschränkt wird. Beispielsweise kann das Reinigungs- und Desinfektionsgerät mindestens eine Authentifizierungsvorrichtung aufweisen, insbesondere beispielsweise einen RFID-Leser. Auch eine andere Art von Authentifizierungsvorrichtung ist denkbar, beispielsweise ein Eingabefeld zur Eingabe eines Codes, ein Kartenleser oder eine ähnliche Art von Authentifizierungsvorrichtung. Mittels der Authentifizierungsvorrichtung kann allgemein eine Authentifizierung des Benutzers durchführbar sein. Unter einer Authentifizierung kann allgemein eine Erkennung eines bestimmten Benutzers verstanden sowie optional eine Abfrage, ob der Benutzer zur Durchführung einer oder mehrerer Handlungen berechtigt ist, beispielsweise allgemein zur Bedienung des Reinigungs- und Desinfektionsgeräts und/oder zur Durchführung des Lernprogramms. Beispielsweise kann das Reinigungs- und Desinfektionsgerät derart eingerichtet sein, dass eine Durchführung des Lernprogramms auf einen vorgegebenen Benutzerkreis begrenzbar ist.

Zusätzlich zu dem mindestens einen Bilddetektor kann das Reinigungs- und Desinfektionsgerät einen oder mehrere weitere Detektoren umfassen, welche eingerichtet sein können, um eine oder mehrere Messgrößen zu erfassen. Beispielsweise kann das Reinigungs- und Desinfektionsgerät in einem Inneren der Reinigungskammer mindestens einen Prozesssensor aufweisen, insbesondere mindestens einen optischen Prozesssensor. Unter einem Prozesssensor wird dabei allgemein eine Vorrichtung verstanden, welche eingerichtet ist, um einen Verschmutzungsgrad des Behälters zu erkennen. Der Prozesssensor kann insbesondere mindestens einen optischen Detektor umfassen. Weiterhin kann der Prozesssensor mindestens eine Beleuchtungseinrichtung umfassen. Das Reinigungs- und Desinfektionsgerät kann beispielsweise eingerichtet sein, um einen Ablauf des Reinigungsprogramms entsprechend des erkannten Verschmutzungsgrads zu verändern, insbesondere eine Zusammensetzung des Reinigungsprogramms und/oder mindestens einen Parameter mindestens eines Reinigungsprogrammschritts des Reinigungsprogramms, beispielsweise eine Dauer der Beaufschlagung mit dem Reinigungsfluid und/oder eine Zusammensetzung des Reinigungsfluids. Beispielsweise kann in regelmäßigen oder unregelmäßigen Abständen mittels des Prozesssensors der Verschmutzungsgrad des Behälters erkannt werden, und es kann anhand des Verschmutzungsgrades und beispielsweise eventueller anhaftender Verschmutzungen entschieden werden, vorzugsweise automatisch, ob das Reinigungsprogramm, beispielsweise eine Beaufschlagung mit dem Reinigungsfluid, fortgesetzt werden soll oder nicht. Für weitere mögliche Ausgestaltungen des Prozesssensors kann beispielsweise auf den oben genannten Stand der Technik verwiesen werden, wobei beispielsweise die aus der Geschirrspültechnik bekannten Sensoriken auch im Rahmen des hier eingesetzten Prozesssensors verwendbar sind. Besonders bevorzugt umfasst der Prozesssensor mindestens einen weiteren optischen Sensor, insbesondere mindestens eine Kamera, welche, beispielsweise wiederum durch Bilderkennung und/oder durch Reflexionsmessungen an mindestens einer Oberfläche des Behälters, den Verschmutzungsgrad bestimmen kann. Das Reinigungs- und Desinfektionsgerät kann weiterhin eingerichtet sein, beispielsweise durch eine entsprechende programmtechnische Einrichtung der mindestens einen Steuerung, um nach Durchführung des Reinigungsprogramms mittels des Bilddetektors mindestens ein Reinigungsergebnis zu erkennen. Beispielsweise kann dies mittels des optional auf der Außenseite des Reinigungs- und Desinfektionsgeräts, beispielsweise auf einer Außenseite der Reinigungskammer des Reinigungs- und Desinfektionsgeräts, angeordneten Bilddetektors erfolgen, sobald beispielsweise die Tür der Reinigungskammer geöffnet wird und/oder sobald der Behälter aus der Reinigungsposition in die Beladungsposition verbracht wird oder verbracht ist. Beispielsweise kann dies bei einer geöffneten Klappe des Reinigungs- und Desinfektionsgeräts erfolgen, beispielsweise bei geöffneter Klappe mit an dieser Klappe befestigter Halterung. Entsprechend kann das Reinigungs- und Desinfektionsgerät beispielsweise eingerichtet sein, um mindestens eine Information über das Reinigungsergebnis an einen Benutzer auszugeben, beispielsweise mindestens eine Information darüber, dass das Reinigungsergebnis korrekt ist oder ausreichend ist, oder eine Information darüber, dass eine ungenügende Reinigung erfolgt ist, beispielsweise begleitet von einer Empfehlung zur Durchführung mindestens eines weiteren Reinigungsprogramms und/oder mindestens eines weiteren Reinigungsprogrammschritts.

Das Reinigungs- und Desinfektionsgerät kann insbesondere mindestens eine Warnvorrichtung zur Ausgabe mindestens einer Warnung an einen Benutzer aufweisen. Diese Warnvorrichtung kann beispielsweise Bestandteil der oben beschriebenen optionalen Mensch-Maschine-Schnittstelle sein. Die Warnvorrichtung kann beispielsweise eingerichtet sein, um die Warnung optisch und/oder akustisch und/oder haptisch und/oder elektronisch auszugeben. Beispielsweise kann die Warnvorrichtung mindestens ein Display und/oder mindestens eine Warnlampe umfassen. Die Reinigungsvorrichtung kann beispielsweise eingerichtet sein, um die Warnung auszugeben, wenn die erkannte Eigenschaft ausgewählt ist aus der Gruppe bestehend aus: der Behälter ist ungeeignet für eine Behandlung in dem Reinigungs- und Desinfektionsgerät; der Behälter wurde nicht erkannt; der Behälter ist verschlossen; der Behälter ist fehlerhaft in eine Halterung des Reinigungs- und Desinfektionsgeräts eingefügt.

In einem weiteren Aspekt der vorliegenden Erfindung wird ein Verfahren zur Behandlung von mindestens einem Behälter für menschliche Ausscheidungen vorgeschlagen. Wie oben ausgeführt, kann dieses Verfahren insbesondere unter Verwendung eines Reinigungs- und Desinfektionsgeräts gemäß der vorliegenden Erfindung durchgeführt werden. Für mögliche Ausgestaltungen des Verfahrens kann dementsprechend auf die obige oder nachfolgende Beschreibung bevorzugte Ausgestaltungen des Reinigungs- und Desinfektionsgeräts verwiesen werden oder umgekehrt. Bei dem Verfahren wird mindestens ein Reinigungsprogramm durchgeführt, beispielsweise ein Reinigungsprogramm gemäß der obigen Beschreibung mit mindestens einem Reinigungsprogrammschritt. In dem Reinigungsprogramm wird der Behälter entleert, insbesondere innerhalb einer Reinigungskammer. Beispielsweise kann dieses Entleeren, wie oben beschrieben, bei einem Schließen der Reinigungskammer erfolgen, beispielsweise durch einen Benutzer und/oder automatisch, und/oder innerhalb der Reinigungskammer und/oder bei einem Verbringen des Behälters von einer Beladungsposition in eine Reinigungsposition. Beispielsweise kann dieses Entleeren durch ein Schwenken des Behälters erfolgen. Der Behälter wird in mindestens eine Reinigungskammer mit mindestens einem Reinigungsfluid beaufschlagt. Dies kann beispielsweise durch mindestens eine Fluidvorrichtung gemäß der obigen Beschreibung erfolgen. Mittels mindestens eines Bilddetektors wird mindestens eine Eigenschaft des Behälters erkannt. Weiterhin wird das Reinigungsprogramm entsprechend der erkannten Eigenschaft beeinflusst.

Wie oben ausgeführt, kann die Eigenschaft insbesondere vor und/oder während eines Einbringens des Behälters in die Reinigungskammer erkannt werden, beispielsweise während eines Schließens mindestens einer Tür der Reinigungskammer, wobei vorzugsweise mindestens eine Halterung für den Behälter mit der Tür verbunden ist.

Das Verfahren kann insbesondere derart durchgeführt werden, dass mindestens ein Bild des Bilddetektors mit einer Mehrzahl von Bildmustern verglichen wird. Insbesondere kann eine Mehrzahl von in einer Steuerung hinterlegten Bildmustern verwendet werden. Entsprechend eines Ergebnisses des Vergleichs kann beispielsweise das Reinigungsprogramm beeinflusst werden. Beispielsweise kann, wie oben ausgeführt, für jedes Bildmuster mindestens eine Art der Beeinflussung hinterlegt sein, wobei diese Art der Beeinflussung gewählt wird, wenn die erkannte Eigenschaft mit dem jeweiligen hinterlegten Bildmuster übereinstimmt.

Wie oben ausgeführt, kann das Verfahren insbesondere derart durchgeführt werden, dass mindestens ein Lernprogramm durchgeführt wird. In dem Lernprogramm kann mindestens ein Behälter mit mindestens einer bekannten Eigenschaft durch einen Benutzer in einen Sichtbereich des Bilddetektors eingebracht werden. Dieses Einbringen des Behälters kann insbesondere mittels einer Halterung erfolgen. Mittels des Bilddetektors kann die Eigenschaft erkannt werden. Durch den Benutzer kann eine der Eigenschaft entsprechende Beeinflussung des Reinigungsprogramms vorgegeben und abgespeichert werden. Diese Vorgabe kann beispielsweise dadurch erfolgen, dass ein Benutzer, beispielsweise mittels einer entsprechenden Tastatur und/oder einer anderen Art von Mensch-Maschine-Schnittstelle, eine Beeinflussung des Reinigungsprogramms auswählt, welche der erkannten Eigenschaft entsprechen soll. Auf diese Weise kann beispielsweise ein Benutzer gezielt bestimmte Eigenschaften und die jeweilige Beeinflussung des Reinigungsprogramms vorgeben. Beispielsweise können bestimmte Arten und/oder Typen von Behältern vorgegeben werden sowie entsprechende Reinigungsprogramme. Wiederum alternativ oder zusätzlich können bestimmte Arten der Befüllung und/oder bestimmte Arten der Verschmutzung vorgegeben werden und entsprechende Reinigungsprogramme hierfür vorgegeben und abgespeichert werden. Allgemein kann durch eine wiederholte Durchführung des Lernprogramms eine Datenbank erzeugt werden, wobei in der Datenbank eine Mehrzahl erkennbarer Eigenschaften sowie zu jeder Eigenschaft mindestens eine durchzuführende Beeinflussung des Reinigungsprogramms hinterlegt werden kann.

Der Bilddetektor wird, wie oben beschrieben, zur Erkennung der mindestens einen Eigenschaft des Behälters eingesetzt. Zusätzlich kann der mindestens eine Bilddetektor jedoch auch zur Erkennung eines oder mehrerer weiterer Parameter eingesetzt werden. Beispielsweise können das Verfahren und/oder das Reinigungs- und Desinfektionsgerät derart eingerichtet sein, dass mittels des Bilddetektors erkannt wird, ob sich mindestens ein Objekt in einem Gefahrenbereich befindet, beispielsweise einem Gefahrenbereich vor einer Öffnung der Reinigungskammer. Beispielsweise können das Verfahren und die Reinigungsvorrichtung derart eingerichtet sein, dass mittels des Bilddetektors mindestens ein Objekt in mindestens einem Gefahrenbereich des Reinigungs- und Desinfektionsgeräts erkannt wird, beispielsweise in einem Schwenkbereich und/oder Bewegungsbereich einer Tür des Reinigungs- und Desinfektionsgeräts. Das Reinigungs- und Desinfektionsgerät kann insbesondere derart eingerichtet sein, dass bei Erkennen eines derartigen Objekts eine Warnung an einen Benutzer ausgegeben wird, beispielsweise mittels der oben beschriebenen Warnvorrichtung. Beispielsweise kann auf diese Weise erkannt werden, dass, bei geöffneter Tür, ein Objekt, beispielsweise eine Hand eines Benutzers, in einem Schwenkbereich und/oder Bewegungsbereich der Tür angeordnet ist, wobei beispielsweise eine Warnung erfolgen kann und/oder wobei ein Schließen der Tür und damit ein Quetschen des Objekts verhindert werden kann.

Das vorgeschlagene Verfahren und die vorgeschlagene Reinigungsvorrichtung weisen gegenüber bekannten Verfahren und Reinigungsvorrichtungen eine Vielzahl von Vorteilen auf. So lässt sich der Bilddetektor insbesondere auf einfache Weise mit einer Kamera realisieren, welche beispielsweise dazu eingerichtet sein kann, eine Beladung des Reinigungs- und Desinfektionsgeräts, mit welcher das Reinigungs- und Desinfektionsgerät beschickt wird oder beschickt werden soll, zu erkennen. Der Bilddetektor kann ein oder mehrere Bilder erzeugen, also beispielsweise ein einzelnes Bild oder eine Sequenz von Bildern, welche in einer Steuerung des Reinigungs- und Desinfektionsgeräts verarbeitet werden können, worauf dementsprechend eine oder mehrere Reaktionen des Reinigungs- und Desinfektionsgeräts eingeleitet werden können.

Der Bilddetektor kann insbesondere außerhalb der Reinigungskammer angeordnet sein, beispielsweise außerhalb oder vor der Reinigungskammer. Der Bilddetektor kann fest mit der Reinigungskammer verbunden sein oder auch als separater Detektor unabhängig hiervon angeordnet sein. Beispielsweise kann der Bilddetektor in einem Rahmen der Reinigungskammer angeordnet werden oder mit einem Rahmen der Reinigungskammer verbunden sein. Insbesondere durch eine Anordnung des Bilddetektors außerhalb eines Innenraums der Reinigungskammer lässt sich bestmöglich eine Verschmutzung des Bilddetektors, beispielsweise einer Optik des Bilddetektors, verhindern. Weiterhin lässt sich hierdurch auch ein Beladebereich der Reinigungskammer überwachen. Zudem sind außerhalb der Reinigungskammer üblicherweise bessere Beleuchtungsverhältnisse vorzufinden, sodass die Bilderkennung durch eine verbesserte Beleuchtung unterstützt werden kann.

Die Kamera kann auf einfache Weise ausgestaltet werden und kann beispielsweise als Autofokuskamera oder auch als Fixfokuskamera ausgestaltet sein. Optional lässt sich, wie oben beschrieben, eine Bilderkennung durch eine Beleuchtung eines Sichtbereichs, beispielsweise eines Erfassungsgebiets, realisieren, damit eine Erkennung beispielsweise auch bei ungünstigen Umgebungslichtverhältnissen möglich wird.

Eine Erkennung der Eigenschaft des Behälters, beispielsweise der Beladung, kann insbesondere vor und/oder während eines Schließens einer Türe der Reinigungskammer erfolgen. Weiterhin kann auf einfache Weise beispielsweise ein Bildvergleich realisiert werden, beispielsweise mit einem oder mehreren Bildmustern in einem Katalog, der beispielsweise in einer Steuerung hinterlegt sein kann.

Die oben beschriebene Möglichkeit der Realisierung eines Lernprogramms bietet im Übrigen eine größtmögliche Flexibilität der vorgeschlagenen Reinigungsvorrichtung und/oder des vorgeschlagenen Verfahrens. Insbesondere lassen sich auf diese Weise ein oder mehrere erkennbare Eigenschaften sowie entsprechende Reaktionen des Reinigungs- und Desinfektionsgeräts als Beeinflussung anlernen: eine bestimmte Art und/oder Form des Behälters; eine bestimmte Beladung des Behälters, beispielsweise eine wässrige oder nichtwässrige Beladung. Erlernte Bildmuster können beispielsweise in einem Katalog, beispielsweise einer Datenbank, aufgenommen werden.

Das Reinigungs- und Desinfektionsgerät kann eingerichtet sein, um zulässige Behälter zu erkennen, wie beispielsweise zulässige Gefäße, Sammelbeutel oder ähnliche Behälter. Bei Erkennung eines unbekannten oder nicht zugelassenen Behälters kann eine Information und/oder insbesondere eine Warnung an einen Benutzer und/oder ein anderes Gerät ausgegeben werden. Das Reinigungs- und Desinfektionsgerät kann eingerichtet sein, dass der Benutzer die Warnung quittieren muss. Das Reinigungs- und Desinfektionsgerät kann weiterhin eingerichtet sein, dass die Erkennung eines unbekannten oder nicht zugelassenen Behälters protokolliert wird, beispielsweise in einem Prozessprotokoll, wobei auch beispielsweise eine Quittierung einer Warnung durch einen Benutzer protokolliert werden kann, gegebenenfalls einschließlich einer Identifikation des Benutzers. Das Reinigungs- und Desinfektionsgerät kann allgemein eingerichtet sein, um ein Protokoll mit Verlaufsdaten des Reinigungsprogramms zu erstellen. Das Quittieren der Warnung kann beispielsweise derart ausgestaltet sein, dass dieses nur mit einer persönlichen Identifikation, beispielsweise durch einen Mitarbeiterausweis mit Chip, einen RFID-Ausweis oder eine andere Art einer Authentifizierungsvorrichtung ermöglicht wird.

Nach Erkennung des eingesetzten Behälters, beispielsweise durch eine Steuerung, kann seitens des Reinigungs- und Desinfektionsgeräts ein Programm vorgeschlagen werden, beispielsweise durch eine optische Hervorhebung einer entsprechenden Programmtaste, eine akustische Ansage eines empfohlenen Programms oder eine Vorauswahl seitens einer Steuerung, welche aktiviert wird, beispielsweise durch ein Schließen der Tür.

Weiterhin können zuverlässig auch Bedienungsfehler beim Einsetzen des Behälters erkannt werden. Beispielsweise lassen sich falsch eingesetzte Behälter erkennen. Auch dies kann unter der Eigenschaft des Behälters subsumierbar sein, sodass die mindestens eine Eigenschaft des Behälters allgemein auch eine Eigenschaft einer Positionierung und/oder Orientierung des Behälters in einer Halterung umfassen kann. Auf diese Weise kann beispielsweise auch erkannt werden, dass ein Behälter eine falsche Position einnimmt, dass ein Deckel nicht abgenommen ist oder eine ähnliche Eigenschaft oder Kombination von Eigenschaften. In diesem Fall kann wiederum ein Hinweis und/oder eine Warnung für einen Benutzer ausgegeben werden, mittels derer auf die Fehlbedienung hingewiesen werden kann.

Allgemein lässt sich mittels des vorgeschlagenen Reinigungs- und Desinfektionsgeräts und des vorgeschlagenen Verfahrens eine Hygienesicherheit erhöhen. Dies ist insbesondere dadurch bedingt, dass Fehlbedienungen, welche typischerweise zu einer verringerten Reinigungsleistung und/oder zu einem verringerten Desinfektionsergebnis führen, zuverlässig vermieden werden können. Weiterhin lassen sich für den Betreiber des Reinigungs- und Desinfektionsgeräts Fehlbedienungen, beispielsweise anhand eines Protokolls, zuverlässig erkennen. Beispielsweise durch Auslesen des Protokolls und/oder des Verlaufsspeichers können Erkenntnisse zu einem Schulungsbedarf beim Personal gewonnen werden.

Auch für den Hersteller des Reinigungs- und Desinfektionsgerätes ergeben sich durch die erfindungsgemäße Ausgestaltung Vorteile. Neben der oben beschriebenen einfachen Implementierung, auch in bestehende Reinigungs- und Desinfektionsgeräte, lässt sich beispielsweise eine Marktbeobachtung durch den Hersteller des Reinigungs- und Desinfektionsgeräts durchführen, indem beispielsweise gesammelte Protokolle ausgewertet werden. Auf diese Weise lassen sich auch entsprechende gesetzliche Vorschriften für Medizinprodukte besser umsetzen. Allgemein können Erkenntnisse zu Produktverbesserungen gewonnen werden, beispielsweise indem ein Benutzerverhalten ausgewertet wird.

Weiterhin lassen sich das Reinigungs- und Desinfektionsgerät und das Verfahren auch derart ausgestalten, dass ein Reinigungsergebnis am Programmende nach einem Öffnen der Tür beurteilt werden kann. Dieses Reinigungsergebnis kann beispielsweise entsprechend auf einer Anzeige, beispielsweise einem Display, oder beispielsweise mittels einer Lampe angezeigt werden. So kann beispielswese eine einfache rot-grün-Leuchte verwendet werden, wobei beispielsweise ein rotes Licht für ein nicht ordnungsgemäßes Reinigungsergebnis aufleuchten kann und ein grünes Licht für ein ordnungsgemäßes Reinigungsergebnis. Es lässt sich dementsprechend ein Reinigungsprogramm anpassen, beispielsweise ein Reinigungsprogramm, welches sich an die bereits abgeschlossene Reinigung desselben Behälters anschließt oder ein Reinigungsprogramm für einen anderen Behälter. So können beispielsweise, entsprechend des zuvor erzielten Reinigungsergebnisses, ein oder mehrere Reinigungsprogrammparameter des nachfolgenden Reinigungsprogramms geändert werden. So lassen sich beispielsweise bei Tropfen auf den Behältern eine Dosierung eines oder mehrerer Zusätze des Reinigungsfluids verändern und/oder mindestens ein Trocknungsschritt verändern, beispielsweise eine Zeitdauer mindestens eines Trocknungsschritts verlängern. Auch andere Ausgestaltungen sind möglich.

Das Reinigungs- und Desinfektionsgerät kann, wie oben ausgeführt, ausgestaltet sein, um mindestens ein Verlaufsprotokoll zu speichern, in welchem beispielsweise Ist-Daten eines oder mehrerer Sensoren und/oder mittels des Bilddetektors erkannte Eigenschaften protokolliert werden können sowie gegebenenfalls weitere Ereignisse. Dieses Verlaufsprotokoll lässt sich beispielsweise in einem Datenspeicher der Steuerung des Reinigungs- und Desinfektionsgeräts und/oder in der Steuerung selbst abspeichern. Das Reinigungs- und Desinfektionsgerät und/oder die Steuerung kann, wie oben ausgeführt, auch mindestens eine Schnittstelle umfassen, sodass beispielsweise eine Übertragung der Daten des Verlaufsprotokolls an ein weiteres Gerät möglich ist, beispielsweise eine Datenfernübertragung an eine Leitwarte und/oder in ein Servicenetz des Herstellers. Auf diese Weise kann das Reinigungs- und Desinfektionsgerät auch zuverlässig in eine zentrale Gerätesteuerung beispielsweise eines Krankenhauses oder eines Pflegeheims eingebunden werden, wobei aus einer Leitwarte heraus, ohne persönliche Präsenz vor Ort, auch ein Reinigungsergebnis und/oder eine korrekte Bedienung und/oder eine korrekte Funktion des Reinigungs- und Desinfektionsgeräts überwacht werden können. Auf diese Weise ist eine erhöhte Prozesssicherheit gegeben.

Weiterhin lässt sich, wie oben beschrieben, auch ein Gefahrenbereich erfindungsgemäß absichern. So lässt sich beispielsweise sicherstellen, dass keine bewegten und/oder unzulässigen Objekte in einem Gefahrenbereich, beispielsweise einem Schwenkbereich einer Tür, angeordnet sind. Werden beispielsweise bestimmte Objekte in diesem Gefahrenbereich erkannt, beispielsweise ein Arm eines Benutzers, ein unzulässiger Behälter oder ähnliche Objekte, so kann beispielsweise ein Schließen der Tür verhindert werden. Erst nach Entfernen derartiger Objekte aus dem Gefahrenbereich kann ein Schließen der Tür freigegeben werden. Insgesamt lässt sich auf diese Weise die Prozesssicherheit erheblich erhöhen, und Unfallgefahren lassen sich vermeiden.

Durch den oben beschriebenen optionalen mindestens einen Prozesssensor, welcher im Inneren der Reinigungskammer angeordnet ist, beispielsweise eine zusätzliche Kamera im Inneren der Reinigungskammer, lässt sich das Reinigungsergebnis zusätzlich überwachen. Mittels dieses Prozesssensors kann beispielsweise ein Reinigungsergebnis während des Ablaufs des Reinigungsprogramms überprüft und/oder überwacht werden. Beispielsweise kann mittels einer Kamera des Prozesssensors, beispielsweise wiederum über eine Bilderkennung, ein Vergleich mit einem oder mehreren vorgegebenen Bildern einer Datenbank durchgeführt werden, um ein Reinigungsergebnis zu überwachen. Insbesondere kann das Reinigungs- und Desinfektionsgerät eingerichtet sein, um während des Ablaufs des Reinigungsprogramms vor einer thermischen Desinfektion ein Reinigungsergebnis zu überprüfen. Auf diese Weise kann beispielsweise verhindert werden, dass aufgrund eines unzureichenden Reinigungsergebnisses noch Verunreinigungen an dem Behälter anhaften und dennoch die thermische Desinfektion gestartet wird, was zu einem Festbacken von Schmutzresten führen könnte. Entsprechend des mittels des Prozesssensors erkannten Reinigungsergebnisses kann das Reinigungs- und Desinfektionsgerät eingerichtet sein, um das Reinigungsprogramm zu verändern. Beispielsweise können ein oder mehrere Prozessparameter während der Behandlung verändert, insbesondere angepasst werden. Beispielsweise kann gegebenenfalls das Reinigungsprogramm und/oder mindestens ein Reinigungsprogrammschritt des Reinigungsprogramms, verlängert werden. Alternativ oder zusätzlich können ein oder mehrere Spülgänge ergänzt werden und/oder es kann eine zusätzliche Komponente in das Reinigungsfluid dosiert werden, beispielsweise eine zusätzliche Prozesschemie.

Das Reinigungs- und Desinfektionsgerät ist allgemein beispielsweise als Frontlader oder auch als Toplader realisierbar. Bei einem Frontlader ist hierbei eine Tür im Frontbereich des Reinigungs- und Desinfektionsgeräts angeordnet, beispielsweise eine Schwenktür. Bei einem Toplader ist diese Tür auf einer Oberseite des Reinigungs- und Desinfektionsgeräts angeordnet.

Der mindestens eine Bilddetektor kann beispielsweise fest mit einem Gehäuse der Reinigungskammer verbunden sein. Alternativ oder zusätzlich kann der mindestens eine Bilddetektor auch als separate Baueinheit neben dem Gehäuse angeordnet werden, wobei dennoch eine Verbindung beispielsweise zu einer Steuerung des Reinigungs- und Desinfektionsgeräts hergestellt werden kann. Diese Verbindung kann beispielsweise drahtgebunden oder auch drahtlos ausgestaltet werden. Allgemein kann der Bilddetektor derart ausgestaltet sein, dass Behälter, bevor diese in die Reinigungskammer eingebracht werden, mindestens einmal einen Sichtbereich des Bilddetektors passieren müssen. Dies kann beispielsweise vor, während oder nach einem Einsetzen des mindestens einen Behälters in einen Halter erfolgen.

Zusammenfassend sind im Rahmen der vorliegenden Erfindung folgende Merkmale besonders bevorzugt:
Ausführungsform 1: Reinigungs- und Desinfektionsgerät zur Behandlung mindestens eines Behälters für menschliche Ausscheidungen, umfassend mindestens eine Reinigungskammer mit mindestens einer Fluidvorrichtung zur Beaufschlagung des Behälters mit mindestens einem Reinigungsfluid, wobei das Reinigungs- und Desinfektionsgerät eingerichtet ist, um mindestens ein Reinigungsprogramm durchzuführen, wobei in dem Reinigungsprogramm der Behälter entleert und mit dem Reinigungsfluid beaufschlagt wird, wobei das Reinigungs- und Desinfektionsgerät weiterhin mindestens einen Bilddetektor aufweist, wobei der Bilddetektor eingerichtet ist, um mindestens eine Eigenschaft des Behälters zu erkennen und wobei das Reinigungs- und Desinfektionsgerät eingerichtet ist, um das Reinigungsprogramm entsprechend der erkannten Eigenschaft zu beeinflussen.
Ausführungsform 2: Reinigungs- und Desinfektionsgerät nach der vorhergehenden Ausführungsform, wobei der Bilddetektor außerhalb der Reinigungskammer angeordnet ist.
Ausführungsform 3: Reinigungs- und Desinfektionsgerät nach der vorhergehenden Ausführungsform, wobei das Reinigungs- und Desinfektionsgerät, insbesondere der Bilddetektor, eingerichtet ist, um die Eigenschaft vor und/oder während eines Einbringens des Behälters in die Reinigungskammer zu erkennen.
Ausführungsform 4: Reinigungs- und Desinfektionsgerät nach einer der beiden vorhergehenden Ausführungsformen, wobei das Reinigungs- und Desinfektionsgerät, insbesondere der Bilddetektor, eingerichtet ist, um die Eigenschaft nach Ablauf eines Reinigungsprogramms zu erkennen.
Ausführungsform 5: Reinigungs- und Desinfektionsgerät nach einer der drei vorhergehenden Ausführungsformen, wobei der Bilddetektor auf einer Außenseite der Reinigungskammer angeordnet ist und wobei eine Eingabeöffhung der Reinigungskammer zumindest teilweise in einem Sichtbereich des Bilddetektors angeordnet ist.
Ausführungsform 6: Reinigungs- und Desinfektionsgerät nach einer der vorhergehenden Ausführungsformen, wobei das Reinigungs- und Desinfektionsgerät eine bewegbare Tür aufweist, insbesondere eine Klappe, wobei mindestens eine Halterung zur Aufnahme des Behälters mit der Tür verbunden ist, wobei der Bilddetektor eingerichtet ist, um die Eigenschaft des Behälters in der Halterung bei geöffneter Tür zu erkennen.
Ausführungsform 7: Reinigungs- und Desinfektionsgerät nach einer der vorhergehenden Ausführungsformen, wobei das Reinigungs- und Desinfektionsgerät eingerichtet ist, um mindestens ein Bild des Behälters zu erfassen und vorzugsweise abzuspeichern.
Ausführungsform 8: Reinigungs- und Desinfektionsgerät nach der vorhergehenden Ausführungsform, wobei das Reinigungs- und Desinfektionsgerät eingerichtet ist, um mindestens eine Zusatzinformation gemeinsam mit dem Bild abzuspeichern.
Ausführungsform 9: Reinigungs- und Desinfektionsgerät nach der vorhergehenden Ausführungsform, wobei die Zusatzinformation ausgewählt ist aus der Gruppe bestehend aus: einer Information über einen Benutzer des Reinigungs- und Desinfektionsgeräts; einer Information über eine Zeit der Erfassung des Bildes, insbesondere einem Zeitstempel.
Ausführungsform 10: Reinigungs- und Desinfektionsgerät nach einer der vorhergehenden Ausführungsformen, wobei das Reinigungs- und Desinfektionsgerät weiterhin mindestens eine Beleuchtungsvorrichtung aufweist, wobei die Beleuchtungsvorrichtung eingerichtet ist, um einen Sichtbereich des Bilddetektors zumindest teilweise zu beleuchten.
Ausführungsform 11: Reinigungs- und Desinfektionsgerät nach einer der vorhergehenden Ausführungsformen, wobei das Reinigungs- und Desinfektionsgerät mindestens eine Halterung zur Aufnahme des Behälters aufweist, wobei die Halterung mindestens eine Beladungsposition außerhalb der Reinigungskammer aufweist, wobei der Behälter durch einen Benutzer in der Beladungsposition in die Halterung einbringbar und aus der Halterung entnehmbar ist, wobei die Halterung weiterhin mindestens eine Reinigungsposition innerhalb der Reinigungskammer aufweist, wobei der Behälter in der Reinigungsposition in der Reinigungskammer angeordnet ist und mit dem Reinigungsfluid beaufschlagbar ist.
Ausführungsform 12: Reinigungs- und Desinfektionsgerät nach der vorhergehenden Ausführungsform, wobei der Bilddetektor eingerichtet ist, um die Eigenschaft in der Beladungsposition zu erkennen.
Ausführungsform 13: Reinigungs- und Desinfektionsgerät nach einer der beiden vorhergehenden Ausführungsformen, wobei das Reinigungs- und Desinfektionsgerät eingerichtet ist, um den Behälter in der Reinigungsposition und/oder bei einem Übergang von der Beladungsposition in die Reinigungsposition zu entleeren, insbesondere in einen Abfluss.
Ausführungsform 14: Reinigungs- und Desinfektionsgerät nach einer der drei vorhergehenden Ausführungsformen, wobei die Halterung zumindest teilweise in einer Tür der Reinigungsvorrichtung angeordnet ist, insbesondere in einer frontseitigen Klappe.
Ausführungsform 15: Reinigungs- und Desinfektionsgerät nach einer der vier vorhergehenden Ausführungsformen, wobei der Behälter bei einem Übergang von der Beladungsposition in die Reinigungsposition geschwenkt wird.
Ausführungsform 16: Reinigungs- und Desinfektionsgerät nach einer der vorhergehenden Ausführungsformen, wobei der Bilddetektor mindestens eine Kamera und mindestens eine Bilderkennungsvorrichtung umfasst.
Ausführungsform 17: Reinigungs- und Desinfektionsgerät nach einer der vorhergehenden Ausführungsformen, wobei die Eigenschaft ausgewählt ist aus der Gruppe bestehend aus: einer Art des Behälters, insbesondere einem Behältertyp und/oder einer Behälterform; einer Befüllung des Behälters mit menschlichen Ausscheidungen, insbesondere einer Füllmenge und/oder einer Art der Befüllung und vorzugsweise einer Erkennung, ob eine wässrige Befüllung vorliegt oder nicht; einem Verschmutzungsgrad des Behälters; einer Art der Verschmutzung des Behälters; einer Eignung des Behälters zur Reinigung in dem Reinigungs- und Desinfektionsgerät; einer Position und/oder Lage des Behälters.
Ausführungsform 18: Reinigungs- und Desinfektionsgerät nach einer der vorhergehenden Ausführungsformen, wobei die Beeinflussung des Reinigungsprogramms ausgewählt ist aus: einer Auswahl eines geeigneten Reinigungsprogramms aus einer Liste von Reinigungsprogrammen; einem Vorschlag eines geeigneten Reinigungsprogramms an einen Benutzer des Reinigungs- und Desinfektionsgeräts; einer Beeinflussung mindestens eines Parameters des Reinigungsprogramms, insbesondere eines Parameters ausgewählt aus der Gruppe bestehend aus einer Dauer mindestens eines Reinigungsprogrammschritts des Reinigungsprogramms, einer Änderung mindestens einer Konzentration mindestens einer Komponente des Reinigungsfluids, insbesondere einer Konzentration mindestens eines Reinigungsmittels und/oder mindestens eines Desinfektionsmittels in dem Reinigungsfluid, einer Temperatur des Reinigungsfluids und/oder einer unterschiedlichen Ansteuerung von verschiedenen Düsen; eine Beaufschlagung des Reinigungsguts aus sich in der Lage ändernden Düsen, insbesondere mindestens einer Hubdrehdüse; einer Verhinderung der Durchführung des Reinigungsprogramms und/oder mindestens eines folgenden Reinigungsprogrammschritts des Reinigungsprogramms; einer Einfügung mindestens eines zusätzlichen Reinigungsprogrammschritts zu dem Reinigungsprogramm; einer Ausgabe mindestens einer Warnung und/oder mindestens eines Hinweises an einen Benutzer.
Ausführungsform 19: Reinigungs- und Desinfektionsgerät nach einer der vorhergehenden Ausführungsformen, wobei das Reinigungs- und Desinfektionsgerät mindestens einen Abfluss mit mindestens einem Geruchsverschluss aufweist, wobei das Reinigungs- und Desinfektionsgerät eingerichtet ist, um den Behälter in dem Reinigungsprogramm in den Abfluss hinein zu entleeren.
Ausführungsform 20: Reinigungs- und Desinfektionsgerät nach der vorhergehenden Ausführungsform, wobei das Reinigungs- und Desinfektionsgerät weiterhin mindestens einen Bypass aufweist, wobei über den Bypass Gas und Dampf aus der Reinigungskammer unter Umgehung des Geruchsverschlusses in den Abfluss ableitbar sind, vorzugsweise unter Druck.
Ausführungsform 21: Reinigungs- und Desinfektionsgerät nach einer der vorhergehenden Ausführungsformen, wobei die Fluidvorrichtung mindestens einen Flüssigkeitstank sowie mindestens eine Düse zur Beaufschlagung des Behälters mit einer Flüssigkeit umfasst, wobei die Fluidvorrichtung weiterhin mindestens einen Dampferzeuger zur Erzeugung von Heißdampf und zur Beaufschlagung des Behälters mit dem Heißdampf aufweist.
Ausführungsform 22: Reinigungs- und Desinfektionsgerät nach einer der vorhergehenden Ausführungsformen, wobei das Reinigungs- und Desinfektionsgerät weiterhin eingerichtet ist, um mindestens ein Lernprogramm durchzuführen, wobei in dem Lernprogramm mindestens ein Behälter mit mindestens einer bekannten Eigenschaft durch einen Benutzer in einen Sichtbereich des Bilddetektors eingebracht wird, insbesondere in einer Halterung, wobei mittels des Bilddetektors die Eigenschaft erkannt wird und durch den Benutzer eine der Eigenschaft entsprechende Beeinflussung des Reinigungsprogramms vorgebbar und in dem Reinigungs- und Desinfektionsgerät speicherbar ist.
Ausführungsform 23: Reinigungs- und Desinfektionsgerät nach der vorhergehenden Ausführungsform, wobei das Reinigungs- und Desinfektionsgerät, insbesondere eine Steuerung des Reinigungs- und Desinfektionsgeräts, mindestens eine Datenbank aufweist, wobei in der Datenbank eine Mehrzahl erkennbarer Eigenschaften sowie zu jeder Eigenschaft jeweils mindestens eine durchzuführende Beeinflussung des Reinigungsprogramms hinterlegt sind.
Ausführungsform 24: Reinigungs- und Desinfektionsgerät nach einer der beiden vorhergehenden Ausführungsformen, wobei das Reinigungs- und Desinfektionsgerät mindestens eine Authentifizierungsvorrichtung aufweist, insbesondere einen RFID-Leser, wobei mittels der Authentifizierungsvorrichtung eine Authentifizierung des Benutzers durchführbar ist und wobei eine Durchführung des Lernprogramms auf einen vorgegebenen Benutzerkreis begrenzbar ist.
Ausführungsform 25: Reinigungs- und Desinfektionsgerät nach einer der vorhergehenden Ausführungsformen, wobei das Reinigungs- und Desinfektionsgerät weiterhin in einem Innenraum der Reinigungskammer mindestens einen Prozesssensor aufweist, insbesondere einen optischen Prozesssensor, wobei der Prozesssensor eingerichtet ist, um einen Verschmutzungsgrad des Behälters zu erkennen, wobei das Reinigungs- und Desinfektionsgerät eingerichtet ist, um einen Ablauf des Reinigungsprogramms entsprechend des erkannten Verschmutzungsgrads zu verändern, insbesondere eine Zusammensetzung des Reinigungsprogramms und/oder mindestens einen Parameter mindestens eines Reinigungsprogrammschritts des Reinigungsprogramms.
Ausführungsform 26: Reinigungs- und Desinfektionsgerät nach einer der vorhergehenden Ausführungsformen, wobei das Reinigungs- und Desinfektionsgerät weiterhin eingerichtet ist, um nach Durchführung des Reinigungsprogramms mittels des Bilddetektors mindestens ein Reinigungsergebnis zu erkennen.
Ausführungsform 27: Reinigungs- und Desinfektionsgerät nach einer der vorhergehenden Ausführungsformen, wobei das Reinigungs- und Desinfektionsgerät derart eingerichtet ist, dass mittels des Bilddetektors mindestens ein Objekt in mindestens einem Gefahrenbereich des Reinigungs- und Desinfektionsgeräts erkannt wird.
Ausführungsform 28: Reinigungs- und Desinfektionsgerät nach einer der vorhergehenden Ausführungsformen, wobei das Reinigungs- und Desinfektionsgerät mindestens eine Warnvorrichtung zur Ausgabe mindestens einer Warnung an einen Benutzer aufweist.
Ausführungsform 29: Reinigungs- und Desinfektionsgerät nach der vorhergehenden Ausführungsform, wobei die Reinigungsvorrichtung eingerichtet ist, um die Warnung auszugeben, wenn die erkannte Eigenschaft ausgewählt ist aus der Gruppe bestehend aus: der Behälter ist ungeeignet; der Behälter wurde nicht erkannt; der Behälter ist verschlossen; der Behälter ist fehlerhaft in eine Halterung eingefügt; mindestens ein unzulässiges Objekt befindet sich in einem Gefahrenbereich des Reinigungs- und Desinfektionsgeräts.
Ausführungsform 30: Verfahren zur Behandlung mindestens eines Behälters für menschliche Ausscheidungen, insbesondere unter Verwendung eines Reinigungs- und Desinfektionsgeräts nach einem der vorhergehenden Ansprüche, wobei mindestens ein Reinigungsprogramm durchgeführt wird, wobei in dem Reinigungsprogramm der Behälter entleert wird, insbesondere innerhalb einer Reinigungskammer, wobei der Behälter in mindestens einer Reinigungskammer mit mindestens einem Reinigungsfluid beaufschlagt wird, wobei mittels mindestens eines Bilddetektors mindestens eine Eigenschaft des Behälters erkannt wird und wobei das Reinigungsprogramm entsprechend der erkannten Eigenschaft beeinflusst wird.
Ausführungsform 31: Verfahren nach der vorhergehenden Ausführungsform, wobei die Eigenschaft vor und/oder während eines Einbringens des Behälters in die Reinigungskammer erkannt wird.
Ausführungsform 32: Verfahren nach einer der beiden vorhergehenden Ausführungsformen, wobei mindestens ein Bild des Bilddetektors mit einer Mehrzahl von Bildmustern verglichen wird, insbesondere mit einer Mehrzahl von in einer Steuerung hinterlegten Bildmustern, wobei entsprechend eines Ergebnisses des Vergleichs das Reinigungsprogramm beeinflusst wird.
Ausführungsform 33: Verfahren nach einer der vorhergehenden, ein Verfahren betreffenden Ausführungsformen, wobei weiterhin mindestens ein Lernprogramm durchgeführt wird, wobei in dem Lernprogramm mindestens ein Behälter mit mindestens einer bekannten Eigenschaft durch einen Benutzer in einen Sichtbereich des Bilddetektors eingebracht wird, insbesondere in einer Halterung, wobei mittels des Bilddetektors die Eigenschaft erkannt wird und wobei durch den Benutzer eine der Eigenschaft entsprechende Beeinflussung des Reinigungsprogramms vorgegeben und abgespeichert wird.
Ausführungsform 34: Verfahren nach der vorhergehenden Ausführungsform, wobei durch wiederholte Durchführung des Lernprogramms eine Datenbank erzeugt wird, wobei in der Datenbank eine Mehrzahl erkennbarer Eigenschaften sowie zu jeder Eigenschaft jeweils mindestens eine durchzuführende Beeinflussung des Reinigungsprogramms hinterlegt werden.
Ausführungsform 35: Verfahren nach einer der vorhergehenden, ein Verfahren betreffenden Ausführungsformen, wobei mittels des Bilddetektors mindestens ein Bild des Behälters aufgenommen wird.
Ausführungsform 36: Verfahren nach der vorhergehenden Ausführungsform, wobei das mindestens eine Bild an mindestens eine externe Datenverarbeitungsvorrichtung übermittelt wird.
Ausführungsform 37: Verfahren nach der vorhergehenden Ausführungsform, wobei die externe Datenverarbeitungsvorrichtung das mindestens eine Bild einer Bildverarbeitung unterzieht.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

### Im Einzelnen zeigen:

- Figur 1: ein erfindungsgemäßes Reinigungs- und Desinfektionsgerät mit einer Halterung in einer Beladungsposition; und
- Figur 2: das Reinigungs- und Desinfektionsgerät gemäß Figur 1 mit der Halterung in einer Reinigungsposition.

### Ausführungsbeispiele

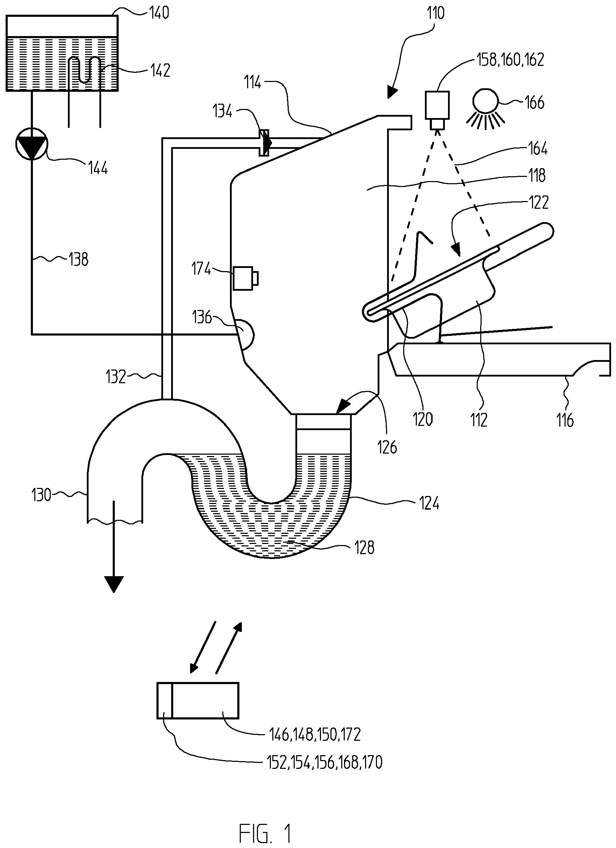

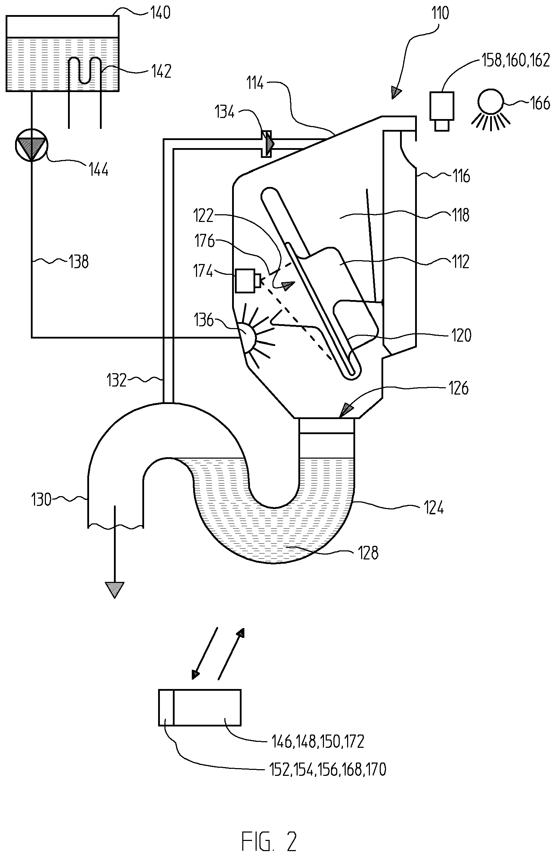

Im Folgenden wird ein Ausführungsbeispiel eines erfindungsgemäßen Reinigungs- und Desinfektionsgeräts 110 zur Behandlung mindestens eines Behälters 112 für menschliche Ausscheidungen in verschiedenen Positionen gezeigt. Dabei stellt Figur 1 eine schematische Schnittdarstellung des Reinigungs- und Desinfektionsgeräts 110 in einer unten noch näher beschriebenen Beladungsposition dar, wohingegen Figur 2 das Ausführungsbeispiel gemäß Figur 1 in einer Reinigungsposition zeigt. Beide Figuren werden im Folgenden gemeinsam erläutert.

Das Reinigungs- und Desinfektionsgerät 110 umfasst eine Reinigungskammer 114 mit einer Tür 116, über welche ein Innenraum 118 der Reinigungskammer 114 zugänglich ist. Im dargestellten Ausführungsbeispiel ist die Tür 116 als Frontklappe ausgestaltet, sodass das Reinigungs- und Desinfektionsgerät 110 allgemein als Frontlader ausgestaltet ist. Auch andere Ausgestaltungen sind jedoch grundsätzlich möglich. Das Öffnen und/oder das Schließen der Tür 116 kann automatisch erfolgen, beispielsweise durch mindestens einen in den Figuren nicht dargestellten Antrieb, oder auch, alternativ oder zusätzlich, durch eine manuelle Bedienung durch einen Anwender.

An einer dem Innenraum 118 zuweisenden Seite der Tür 116 ist eine Halterung 120 angeordnet. Diese Halterung 120 kann beispielsweise ein Drahtgestänge und/oder einen Drahtkorb oder eine andere Art von Halterung 120 umfassen. In Figur 1 ist die Tür 116 in einem geöffneten Zustand dargestellt, und die Halterung 120 befindet sich in einer Beladungsposition. In dieser Beladungsposition kann der Behälter 112, im dargestellten Ausführungsbeispiel ein Steckbecken, in die Halterung 120 derart eingesetzt werden, dass ein Inhalt des Behälters 112 nicht ausfließen kann. Beispielsweise kann dies dadurch erfolgen, dass eine Öffnung 122 des Behälters 112 nach oben oder schräg nach oben weist. In Figur 2 ist die Tür 116 hingegen in einer geschlossenen Position dargestellt, und die Halterung 120 befindet sich in einer Reinigungsposition. In dieser Reinigungsposition weist vorzugsweise die Öffnung 122 des Behälters 112 nach unten oder zumindest schräg nach unten, sodass der Inhalt des Behälters 112 während und/oder nach dem Schließen der Tür 116 in den Innenraum 118 der Reinigungskammer 114 ausfließen kann. Am Boden der Reinigungskammer 114 ist ein Abfluss 124 mit einer entsprechend groß dimensionierten Abflussöffnung 126 von beispielsweise mehr als 30 mm Durchmesser oder Äquivalentdurchmesser, vorzugsweise von mindestens 50 mm oder sogar mindestens 70 mm, oder sogar 100 mm oder mehr vorgesehen, durch welchen größere Abfallmengen entsorgt werden können. Der Abfluss 124 ist vorzugsweise mit einem Geruchsverschluss 128 versehen, beispielsweise einem Siphonbogen, und ist vorzugsweise mit einem oder mehreren Abflussrohren 130 verbunden. Optional kann mindestens ein Bypass 132 vorgesehen sein, welcher den Innenraum 118 der Reinigungskammer 114 unter Umgehung des Geruchsverschlusses 128 mit dem Abfluss 124 und/oder dem Abflussrohr 130 verbindet. In diesem Bypass 132 kann mindestens ein Ventil 134, beispielsweise ein Absperrventil und/oder ein Rückschlagventil, vorgesehen sein. Über den Bypass 132 können beispielsweise Dampf und/oder Gase aus dem Innenraum 118 in den Abfluss 124 zwangsverdrängt werden, beispielsweise über mindestens einen in den Figuren nicht dargestellten Druckstutzen zur Reinigungskammer 114, über welchen beispielsweise Luft in die Reinigungskammer 114 eingepresst werden kann, um den Behälter 112 zu kühlen und/oder um Dampf in den Abfluss 124 zu verdrängen.

In der Reinigungskammer 114 ist mindestens eine Fluidvorrichtung 136 vorgesehen. Diese Fluidvorrichtung 136 kann beispielsweise eine oder mehrere Düsen umfassen, welche über mindestens eine Zuleitung 138 mit mindestens einem Reinigungsfluid beaufschlagt werden kann. Die Zuleitung 138 kann beispielsweise aus einem oder mehreren Fluidtanks 140 gespeist werden. Optional kann der mindestens eine Fluidtank 140 mindestens eine Heizvorrichtung 142 aufweisen, um das Reinigungsfluid ganz oder teilweise zu erwärmen. Um das Reinigungsfluid der mindestens einen Düse unter Druck zuzuführen, kann in dem Fluidtank 140 und/oder in der Zuleitung 138 weiterhin mindestens eine Pumpe 144 vorgesehen sein. Weiterhin kann die Fluidvorrichtung 136 und/oder das Reinigungs- und Desinfektionsgerät 110 mindestens einen Dampferzeuger aufweisen, welcher in den Figuren 1 und 2 nicht dargestellt ist und mittels dessen, beispielsweise nach einer Fluidbeaufschlagung, eine Desinfektion des Behälters 118 mit Heißdampf erfolgen kann, beispielsweise über die Zuleitung 138 und/oder eine getrennte Zuleitung.

Das Reinigungs- und Desinfektionsgerät 110 weist weiterhin mindestens eine Steuerung 146 auf, welche in den Figuren lediglich schematisch dargestellt ist. Diese Steuerung 146 kann beispielsweise mindestens eine Datenverarbeitungsvorrichtung 148 mit optional mindestens einem Datenspeicher 150 umfassen. Die Steuerung 146 ist eingerichtet, um mindestens ein Reinigungsprogramm des Reinigungs- und Desinfektionsgeräts 110 durchzuführen. Das Reinigungsprogramm kann beispielsweise automatisch oder auch durch eine Aktion eines Anwenders gestartet werden, beispielsweise durch ein Schließen der Tür 116 und/oder ein Betätigen eines Knopfes und/oder durch Betätigen eines Schalters, beispielsweise eines Fußschalters. Dementsprechend kann die Steuerung 146 beispielsweise auf eines, mehrere oder alle der oben genannten Elemente einwirken und eingerichtet sein, um aktuelle Werte eines oder mehrerer Reinigungsprogrammparameter einzustellen. Beispielsweise können die Pumpe 144 und/oder die Heizvorrichtung 142 und/oder ein Mechanismus zum Schließen der Tür 116 und/oder ein oder mehrere Ventile und/oder ein Betrieb des Dampferzeugers durch die Steuerung 146 gesteuert werden. Auf diese Weise kann beispielsweise ein Reinigungsprogramm durchgeführt werden, welches, nach einem Einsetzen des Behälters 112 durch einen Anwender in die Halterung 120, ein Schließen der Tür 116 und dabei ein Entleeren des Behälters 112 vorsieht. Bei dem Entleeren kann beispielsweise ein Inhalt des Behälters 112 vollständig oder teilweise in den Abfluss 124 entsorgt werden. Anschließend kann das Reinigungsprogramm ein oder mehrere Spülschritte umfassen, in welchen der Behälter 112 einseitig oder von mehreren Seiten mit dem Reinigungsfluid beaufschlagt wird, beispielsweise indem ein Inneres des Behälters 112 ausgespritzt wird. Anschließend können optional eine oder mehrere Desinfektionsschritte durchgeführt werden, bei welchen eine chemische und/oder thermische Desinfektion erfolgen kann. Beispielsweise kann eine chemische Desinfektion unter Besprühen und/oder Bespritzen mit einem Reinigungsfluid mit mindestens einem Desinfektionsmittelzusatz erfolgen. Eine thermische Desinfektion kann beispielsweise, wie oben ausgeführt, unter Verwendung mindestens eines Dampferzeugers erfolgen, bei welchem der Behälter 112 mit Heißdampf beaufschlagt wird. Anschließend können ein oder mehrere Trocknungsschritte durchgeführt werden. Während der Trocknungsschritte kann auch beispielsweise Zuluft in die Reinigungskammer 114 eingeblasen oder eingepresst werden, wobei beispielsweise Heißdampf oder Feuchtigkeit aus der Reinigungskammer 114 über den Bypass 132 in den Abfluss 124 verdrängt werden können. Anschließend kann ein Öffnen der Tür 116 erfolgen, oder es kann eine Freigabe an einer Türöffnung erfolgen. Die Steuerung 146 kann eine oder mehrere Schnittstellen 152 umfassen, beispielsweise ein oder mehrere Mensch-Maschine-Schnittstellen und/oder eine oder mehrere Schnittstellen 152 zur Verbindung des Reinigungs- und Desinfektionsgeräts 110 mit einem oder mehreren weiteren Geräten und/oder einem oder mehreren Datennetzwerken. Insbesondere kann die Schnittstelle mindestens eine Anzeigevorrichtung 154 umfassen, beispielswese ein oder mehrere Displays und/oder ein oder mehrere optische und/oder akustische Anzeigeelemente, wie beispielsweise eine oder mehrere gegebenenfalls farbige Lampen. Beispielsweise können eine grüne Lampe und eine rote Lampe vorgesehen sein. Auch eine akustische Ausgabe kann vorgesehen sein. Weiterhin kann eine Mensch-Maschine-Schnittstelle in Form mindestens eines Bedienelements 156 vorgesehen sein, beispielsweise mindestens einer Tastatur und/oder mindestens eines Knopfes oder Schalters. Die Anzeigevorrichtung 154 und das Bedienelement 156 können auch ganz oder teilweise zusammengefasst sein, beispielsweise in Form eines Touchscreens.

Weiterhin umfasst das Reinigungs- und Desinfektionsgerät 110 gemäß der vorliegenden Erfindung mindestens einen Bilddetektor 158. Insbesondere kann der mindestens eine Bilddetektor 158, wie oben ausgeführt, mindestens eine Kamera 160 umfassen. Weiterhin kann der Bilddetektor 158 mindestens eine Bilderkennungsvorrichtung 162 umfassen. Diese Bilderkennungsvorrichtung 162 ist in den Figuren 1 und 2 symbolisch am Ort der Kamera 160 eingezeichnet. Alternativ oder zusätzlich kann die Bilderkennungsvorrichtung 162 jedoch auch ganz oder teilweise Bestandteil der Steuerung 146 sein, beispielsweise indem die Datenverarbeitungsvorrichtung 148 programmtechnisch zur Durchführung mindestens eines Bilderkennungsprogramms eingerichtet ist. Die Kamera 160 kann beispielsweise direkt oder indirekt mit der Steuerung 146 verbunden sein. Alternativ oder zusätzlich kann die Bilderkennungsvorrichtung 162 jedoch auch ganz oder teilweise am Ort der Kamera 160 angeordnet sein, beispielsweise in Form eines Mikrocontrollers, und/oder kann ganz oder teilweise in die Kamera 160 integriert sein. Der Bilddetektor 158, insbesondere die Kamera 160, ist im dargestellten Ausführungsbeispiel vorzugsweise ganz oder teilweise außerhalb des Innenraums 118 der Reinigungskammer 114 angeordnet, beispielsweise auf einer Außenseite des Gehäuses der Reinigungskammer 114. Dabei kann die Kamera 160 beispielsweise direkt oder indirekt mit der Reinigungskammer 114 verbunden sein oder auch separat angeordnet sein.

Der Bilddetektor 158 ist vorzugsweise derart angeordnet, dass während oder nach dem Einbringen des Behälters 112 in die Halterung 120 der Behälter 112 mindestens einmal einen Sichtbereich 164 des Bilddetektors 158 passiert. Vorzugsweise ist der Bilddetektor 158 derart ausgestaltet und/oder ausgerichtet, dass der Behälter 112, wenn dieser in der in Figur 1 dargestellten Beladungsposition in die Halterung 120 eingefügt ist, im Sichtbereich 164 angeordnet ist, vorzugsweise derart, dass die Öffnung 122 des Behälters 112 in dem Sichtbereich 164 liegt. Insbesondere können auf diese Weise eine Befüllung und/oder eine Verunreinigung des Behälters 112 erkannt werden. Allgemein ist das Reinigungs- und Desinfektionsgerät eingerichtet, um mittels des Bilddetektors 158 mindestens eine Eigenschaft des Behälters 112 zu erkennen, beispielsweise eine Art und/oder einen Typ des Behälters 112 und/oder eine Befüllung des Behälters 112 und/oder eine Positionierung des Behälters 112 und/oder eine Verunreinigung des Behälters 112. Zusätzlich kann das Reinigungs- und Desinfektionsgerät 110 auch eingerichtet sein, um mittels des Bilddetektors 158 unzulässige Objekte im Sichtbereich 164 zu detektieren, beispielsweise einen Arm eines Anwenders. Zur Unterstützung der Bilderkennung und/oder zur Unterstützung der Erkennung der mindestens einen Eigenschaft kann der Bilddetektor 158, wie in den Figuren 1 und 2 dargestellt, weiterhin mindestens eine Beleuchtungsvorrichtung 166 aufweisen, beispielsweise eine Beleuchtungsvorrichtung, welche eingerichtet ist, um den Sichtbereich 164 des Bilddetektors 158 vollständig oder teilweise auszuleuchten und dadurch beispielsweise einen Kontrast und/oder eine Helligkeit eines mittels des Bilddetektors 158 erfassten Bildes zu verbessern.

Entsprechend der mindestens einen erkannten Eigenschaft kann das Reinigungs- und Desinfektionsgerät 110 mindestens ein Reinigungsprogramm beeinflussen. Beispielsweise kann das Reinigungsprogramm auf diese Weise auf die Art und/oder den Typ des Behälters 112 angepasst werden und/oder auf eine Art und/oder Menge einer Befüllung des Behälters 112 und/oder auf eine Art und/oder Menge einer Verunreinigung des Behälters 112. Beispielsweise können Programmdauern eines oder mehrerer der oben genannten Spülschritte auf den Grad der Verunreinigung angepasst werden. Entsprechend der Art der Verunreinigung können auch ein oder mehrere Zusätze zu dem Reinigungsfluid gezielt gewählt werden, beispielsweise ein Zusatz mindestens eines Salbenreinigers zur Entfernung von Salbenresten. Weiterhin kann beispielsweise auch eine Anzahl und/oder eine Auswahl an Düsen der Fluidvorrichtung 136 und/oder eine Ausrichtung einer oder mehrerer Düsen entsprechend dem erkannten Typ des Behälters 112 gewählt werden. Weiterhin können nicht zugelassene Behälter 112 erkannt und beispielsweise mittels der Anzeigevorrichtung 154 und/oder einer Warnvorrichtung 168 entsprechende Warnungen an einen Anwender ausgegeben werden, wenn beispielsweise eine Art und/oder ein Typ des Behälters 112 nicht erkannt wird oder als unzulässig erkannt wird. Die Steuerung 146, insbesondere die Schnittstelle 152, kann insbesondere mindestens eine Authentifizierungsvorrichtung 170 umfassen, beispielsweise ein Eingabefeld zur Eingabe eines Benutzercodes und/oder einen Kartenleser zum Lesen eines Benutzerausweises und/oder einen RFID-Leser zum Lesen eines Benutzerchips, sodass ein Anwender des Reinigungs- und Desinfektionsgeräts 110 erkannt werden kann. Entsprechend kann beispielsweise protokolliert werden, wenn ein Anwender eine Warnung quittiert. Allgemein kann die Steuerung 146 beispielsweise eingerichtet sein, um ein Protokoll der Reinigung des Behälters 112 zu erstellen und beispielsweise in dem Datenspeicher 150 abzuspeichern.

In dem Datenspeicher 150 kann beispielsweise weiterhin auch mindestens eine Datenbank enthalten sein, in welcher beispielsweise einer oder mehreren erkannten Eigenschaften des Behälters 112 jeweils ein oder mehrere Beeinflussungen des Reinigungsprogramms zugeordnet sind, also beispielsweise jeweils ein oder mehrere zu verwendende Datensätze mit jeweils einem oder mehreren Reinigungsprogrammparametern. Entsprechend der erkannten Eigenschaft kann das Reinigungs- und Desinfektionsgerät 110 die Beeinflussung beispielsweise automatisch vornehmen oder kann eine Beeinflussung dahingehend vornehmen, dass einem Anwender, beispielsweise mittels der Anzeigevorrichtung 154, mindestens eine Beeinflussung, beispielsweise mindestens ein bestimmtes Reinigungsprogramm, vorgeschlagen wird, wobei der Anwender auch beispielsweise eine Auswahl aus mehreren vorgeschlagenen Reinigungsprogrammen treffen kann. Diese Auswahl kann beispielsweise mittels eines oder mehrerer Bedienelemente 156 erfolgen und kann beispielsweise wiederum auch protokolliert werden.

Weiterhin kann das Reinigungs- und Desinfektionsgerät 110, beispielsweise die Steuerung 146, auch eingerichtet sein, um mindestens ein Lernprogramm durchzuführen. Auch dieses Lernprogramm kann beispielsweise wiederum mittels der Authentifizierungsvorrichtung 170 initiiert werden, sodass beispielsweise das Lernprogramm lediglich von einem vorgegebenen Anwenderkreis durchgeführt werden kann. Auf diese Weise kann ein Anwender beispielsweise nacheinander mehrere unterschiedliche Behälter 112 und/oder unterschiedlich befüllte Behälter 112 und/oder unterschiedlich verschmutzte Behälter 112 in die Halterung 120 und/oder auf andere Weise in den Sichtbereich 164 einbringen und dementsprechend Beeinflussungen des Reinigungsprogramms vorgeben. So kann beispielsweise ein Anwender für eine bestimmte Art der Befüllung und/oder für eine bestimmte Art von Verschmutzung und/oder für einen bestimmten Typ des Behälters 112 ein bestimmtes Reinigungsprogramm vorgeben. Dieses Reinigungsprogramm kann beispielsweise in einer Datenbank 172 in dem Datenspeicher 150 abgespeichert sein. In einem späteren Betrieb kann beispielsweise eine automatische Auswahl einer Beeinflussung entsprechend dieser Datenbank erfolgen.

Das Reinigungs- und Desinfektionsgerät 110 kann weiterhin eingerichtet sein, um, beispielsweise wiederum mittels des Bilddetektors 158, ein Reinigungsergebnis nach Durchführung des Reinigungsprogramms, optional nach Öffnung der Tür 116, zu erfassen und optional zu bewerten, beispielsweise wiederum in den in Figur 1 gezeigten Beladungspositionen. Entsprechend kann das Reinigungsergebnis beispielsweise protokolliert werden und/oder über die Anzeigevorrichtung 154 dem Anwender zur Kenntnis gebracht werden und/oder über die Warnvorrichtung 168 eine entsprechende Warnung ausgegeben werden. Auch eine automatische Beeinflussung eines oder mehrerer Reinigungsprogrammparameter nachfolgender Reinigungsprogramme kann erfolgen.

Weiterhin kann das Reinigungs- und Desinfektionsgerät 110, wie in den Figuren 1 und 2 ebenfalls als Option dargestellt, einen oder mehrere Prozesssensoren 174 umfassen, welche beispielsweise im Innenraum 118 des Reinigungs- und Desinfektionsgeräts 110 angeordnet sein können. Dieser mindestens eine Prozesssensor 174 kann beispielsweise wiederum mindestens eine Kamera und/oder eine andere Art von Sensor umfassen, beispielsweise einen optischen Sensor. Auch andere Sensoren sind grundsätzlich möglich. Beispielsweise kann mindestens ein optischer Sensor vorgesehen sein, welcher mindestens eine Reflexionsmessung an mindestens einer Oberfläche des Behälters 112 durchführen kann. Auf diese Weise können anhaftende Verunreinigungen an dieser Oberfläche detektierbar sein. Der Prozesssensor 174 kann beispielsweise derart ausgestaltet sein, dass dieser einen Sichtbereich (Bezugsziffer 176 in Figur 2) aufweist, welcher die Öffnung 122 des Behälters 112 in der in Figur 2 gezeigten Reinigungsposition zumindest teilweise abdeckt, sodass beispielsweise mindestens eine Innenoberfläche des Behälters 112 mittels des Prozesssensors 174 erfasst und dort beispielsweise ein Reinigungsergebnis detektiert werden kann. Wie der Bilddetektor 158 kann beispielsweise auch der Prozesssensor 174 mit der Steuerung 176 verbunden sein.

### Bezugszeichenliste

- 110: Reinigungs- und Desinfektionsgerät
- 112: Behälter
- 114: Reinigungskammer
- 116: Tür
- 118: Innenraum
- 120: Halterung
- 122: Öffnung
- 124: Abfluss
- 126: Abflussöffnung
- 128: Geruchsverschluss
- 130: Abflussrohr
- 132: Bypass
- 134: Ventil
- 136: Fluidvorrichtung
- 138: Zuleitung
- 140: Fluidtank
- 142: Heizvorrichtung
- 144: Pumpe
- 146: Steuerung
- 148: Datenverarbeitungsvorrichtung
- 150: Datenspeicher
- 152: Schnittstelle
- 154: Anzeigevorrichtung
- 156: Bedienelement
- 158: Bilddetektor
- 160: Kamera
- 162: Bilderkennungsvorrichtung
- 164: Sichtbereich
- 166: Beleuchtungsvorrichtung
- 168: Warnvorrichtung
- 170: Authentifizierungsvorrichtung
- 172: Datenbank
- 174: Prozesssensor
- 176: Sichtbereich Prozesssensor

## Patentansprüche

1. Reinigungs- und Desinfektionsgerät (110) zur Behandlung mindestens eines Behälters (112) für menschliche Ausscheidungen, umfassend mindestens eine Reinigungskammer (114) mit mindestens einer Fluidvorrichtung (136) zur Beaufschlagung des Behälters (112) mit mindestens einem Reinigungsfluid, wobei das Reinigungs- und Desinfektionsgerät (110) eingerichtet ist, um mindestens ein Reinigungsprogramm durchzuführen, wobei das Reinigungs- und Desinfektionsgerät (110) mindestens eine Steuerung (146) aufweist, wobei die Steuerung (146) mindestens eine Datenverarbeitungsvorrichtung (148) umfasst, welche programmtechnisch eingerichtet ist, um das Reinigungsprogramm zu steuern, wobei in dem Reinigungsprogramm der Behälter (112) automatisch entleert und mit dem Reinigungsfluid beaufschlagt wird, wobei das Reinigungs- und Desinfektionsgerät (110) eingerichtet ist, um bei einer Entleerung den Behälter (112) von einer Transportposition, in welcher ein Inhalt in dem Behälter (112) verbleibt, in mindestens eine Entleerungsposition zu verbringen, in welcher ein möglicher Inhalt des Behälters (112) aus dem Behälter (112) auslaufen kann, wobei der Behälter derart geschwenkt und/oder gekippt wird, dass mindestens eine Öffnung (122) des Behälters (112) derart nach unten weist, dass ein Auslaufen des Inhalts erfolgt, wobei das Reinigungs- und Desinfektionsgerät (110) mindestens einen Abfluss (124) mit mindestens einem Geruchsverschluss (128) aufweist, wobei das Reinigungs- und Desinfektionsgerät (110) eingerichtet ist, um den Behälter (112) in dem Reinigungsprogramm in den Abfluss (124) hinein zu entleeren, wobei das Reinigungs- und Desinfektionsgerät (110) weiterhin mindestens einen Bilddetektor (158) aufweist, wobei der Bilddetektor (158) eingerichtet ist, um mindestens eine Eigenschaft des Behälters (112) zu erkennen und wobei das Reinigungs- und Desinfektionsgerät (110) eingerichtet ist, um das Reinigungsprogramm entsprechend der erkannten Eigenschaft zu beeinflussen, wobei der Bilddetektor (158) außerhalb der Reinigungskammer (114) angeordnet ist und eingerichtet ist, um die Eigenschaft vor und/oder während eines Einbringens des Behälters (112) in die Reinigungskammer (114) zu erkennen, wobei das Reinigungs- und Desinfektionsgerät (110) mindestens eine Halterung (120) zur Aufnahme des Behälters (112) aufweist, wobei die Halterung (120) mindestens eine Beladungsposition außerhalb der Reinigungskammer (114) aufweist, wobei der Behälter (112) durch einen Anwender in der Beladungsposition in die Halterung (120) einbringbar und aus der Halterung (120) entnehmbar ist, wobei die Halterung (120) weiterhin mindestens eine Reinigungsposition innerhalb der Reinigungskammer (114) aufweist, wobei der Behälter (112) in der Reinigungsposition in der Reinigungskammer (114) angeordnet ist und mit dem Reinigungsfluid beaufschlagbar ist.

2. Reinigungs- und Desinfektionsgerät (110) nach dem vorhergehenden Anspruch, wobei der Bilddetektor (158) mindestens eine Kamera (160) umfasst.

3. Reinigungs- und Desinfektionsgerät (110) nach einem der vorhergehenden Ansprüche, wobei das Reinigungs- und Desinfektionsgerät (110) eine bewegbare Tür (116) aufweist, wobei mindestens eine Halterung (120) zur Aufnahme des Behälters (112) mit der Tür (116) verbunden ist, wobei der Bilddetektor (158) eingerichtet ist, um die Eigenschaft des Behälters (112) in der Halterung (120) bei geöffneter Tür (116) zu erkennen.

4. Reinigungs- und Desinfektionsgerät (110) nach einem der vorhergehenden Ansprüche, wobei der Bilddetektor (158) eingerichtet ist, um die Eigenschaft in der Beladungsposition zu erkennen.

5. Reinigungs- und Desinfektionsgerät (110) nach einem der vorhergehenden Ansprüche, wobei das Reinigungs- und Desinfektionsgerät (110) weiterhin eingerichtet ist, um mindestens ein Lernprogramm durchzuführen, wobei die mindestens eine Steuerung (146) des Reinigungs- und Desinfektionsgeräts (110) programmtechnisch eingerichtet ist, um dieses Lernprogramm zu ermöglichen, wobei in dem Lernprogramm mindestens ein Behälter (112) mit mindestens einer bekannten Eigenschaft durch einen Anwender in einen Sichtbereich (164) des Bilddetektors (158) eingebracht wird, wobei mittels des Bilddetektors (158) die Eigenschaft erkannt wird und durch den Anwender eine der Eigenschaft entsprechende Beeinflussung des Reinigungsprogramms vorgebbar und in dem Reinigungs- und Desinfektionsgerät (110) abspeicherbar ist.

6. Reinigungs- und Desinfektionsgerät (110) nach einem der vorhergehenden Ansprüche, wobei das Reinigungs- und Desinfektionsgerät (110) weiterhin in einem Innenraum (118) der Reinigungskammer (114) mindestens einen Prozesssensor (174) aufweist, wobei der Prozesssensor (174) eingerichtet ist, um einen Verschmutzungsgrad des Behälters (112) zu erkennen, wobei das Reinigungs- und Desinfektionsgerät (110) eingerichtet ist, um einen Ablauf des Reinigungsprogramms entsprechend des erkannten Verschmutzungsgrads zu verändern.

7. Reinigungs- und Desinfektionsgerät (110) nach einem der vorhergehenden Ansprüche, wobei das Reinigungs- und Desinfektionsgerät (110) weiterhin eingerichtet ist, um nach Durchführung des Reinigungsprogramms mittels des Bilddetektors (158) mindestens ein Reinigungsergebnis zu erkennen.

8. Reinigungs- und Desinfektionsgerät (110) nach einem der vorhergehenden Ansprüche, wobei das Reinigungs- und Desinfektionsgerät (110) mindestens eine Warnvorrichtung (168) zur Ausgabe mindestens einer Warnung an einen Anwender aufweist.

9. Reinigungs- und Desinfektionsgerät (110) nach einem der vorhergehenden Ansprüche, wobei das Reinigungs- und Desinfektionsgerät (110) derart eingerichtet ist, dass mittels des Bilddetektors (158) mindestens ein Objekt in mindestens einem Gefahrenbereich des Reinigungs- und Desinfektionsgeräts (110) erkannt wird.

10. Verfahren zur Behandlung mindestens eines Behälters (112) für menschliche Ausscheidungen, unter Verwendung eines Reinigungs- und Desinfektionsgeräts (110) nach einem der vorhergehenden Ansprüche, wobei mindestens ein Reinigungsprogramm durchgeführt wird, wobei in dem Reinigungsprogramm der Behälter (112) entleert wird, wobei der Behälter (112) in mindestens einer Reinigungskammer (114) mit mindestens einem Reinigungsfluid beaufschlagt wird, wobei mittels mindestens eines Bilddetektors (158) mindestens eine Eigenschaft des Behälters (112) erkannt wird und wobei das Reinigungsprogramm entsprechend der erkannten Eigenschaft beeinflusst wird.

11. Verfahren nach dem vorhergehenden Anspruch, wobei mittels des Bilddetektors (158) mindestens ein Bild des Behälters (112) aufgenommen wird.

## Claims

1. Cleaning and disinfecting apparatus (110) for treating at least one container (112) for human excretions, comprising at least one cleaning chamber (114) with at least one fluid device (136) for applying at least one cleaning fluid to the container (112), wherein the cleaning and disinfecting apparatus (110) is configured to carry out at least one cleaning program, wherein the cleaning and disinfecting apparatus (110) has at least one control (146), wherein the control (146) comprises at least one data processing device (148) which is configured by program-technical means to control the cleaning program, wherein, in the cleaning program, the container (112) is emptied automatically and the cleaning fluid is applied thereto, wherein, when emptying, the cleaning and disinfecting apparatus (110) is configured to move the container (112) from a transport position, in which a content remains in the container (112), into at least one emptying position, in which a possible content of the container (112) can flow out of the container (112), wherein the container is pivoted and/or tilted in such a way that at least one opening (122) of the container (112) points downward in such a way that the content flows out, wherein the cleaning and disinfecting apparatus (110) has at least one outflow (124) with at least one odor trap (128), wherein the cleaning and disinfecting apparatus (110) is configured to empty the container (112) into the outflow (124) during the cleaning program, wherein the cleaning and disinfecting apparatus (110) furthermore has at least one image detector (158), wherein the image detector (158) is configured to identify at least one property of the container (112) and wherein the cleaning and disinfecting apparatus (110) is configured to influence the cleaning program in accordance with the identified property, wherein the image detector (158) is arranged outside of the cleaning chamber (114) and configured to identify the property prior to and/or during an insertion of the container (112) into the cleaning chamber (114), wherein the cleaning and disinfecting apparatus (110) has at least one holder (120) for holding the container (112), wherein the holder (120) has at least one loading position outside of the cleaning chamber (114), wherein, in the loading position, the container (112) is introducible into the holder (120) and removable from the holder (120) by a user, wherein the holder (120) furthermore has at least one cleaning position within the cleaning chamber (114), wherein the container (112) is arranged in the cleaning chamber (114) in the cleaning position and the cleaning fluid is applicable thereto.

2. Cleaning and disinfecting apparatus (110) according to the preceding claim, wherein the image detector (158) comprises at least one camera (160).

3. Cleaning and disinfecting apparatus (110) according to one of the preceding claims, wherein the cleaning and disinfecting apparatus (110) has a movable door (116), wherein at least one holder (120) for holding the container (112) is connected to the door (116), wherein the image detector (158) is configured to identify the property of the container (112) in the holder (120) when the door (116) is open.

4. Cleaning and disinfecting apparatus (110) according to one of the preceding claims, wherein the image detector (158) is configured to identify the property in the loading position.

5. Cleaning and disinfecting apparatus (110) according to one of the preceding claims, wherein the cleaning and disinfecting apparatus (110) is furthermore configured to carry out at least one learning program, wherein the at least one control (146) of the cleaning and disinfecting apparatus (110) is configured by program-technical means to enable this learning program, wherein, in the learning program, at least one container (112) with at least one known property is introduced into a visual range (164) of the image detector (158) by a user, wherein the property is identified by means of the image detector (158) and an influencing of the cleaning program corresponding to the property is specifiable by the user and storable in the cleaning and disinfecting apparatus (110).

6. Cleaning and disinfecting apparatus (110) according to one of the preceding claims, wherein the cleaning and disinfecting apparatus (110) furthermore has at least one process sensor (174) in an interior (118) of the cleaning chamber (114), wherein the process sensor (174) is configured to identify a degree of soiling of the container (112), wherein the cleaning and disinfecting apparatus (110) is configured to modify a sequence of the cleaning program in accordance with the identified degree of soiling.

7. Cleaning and disinfecting apparatus (110) according to one of the preceding claims, wherein the cleaning and disinfecting apparatus (110) is furthermore configured to identify at least one cleaning result by means of the image detector (158) after carrying out the cleaning program.

8. Cleaning and disinfecting apparatus (110) according to one of the preceding claims, wherein the cleaning and disinfecting apparatus (110) has at least one warning device (168) for outputting at least one warning to a user.

9. Cleaning and disinfecting apparatus (110) according to one of the preceding claims, wherein the cleaning and disinfecting apparatus (110) is configured in such a way that at least one object in at least one danger area of the cleaning and disinfecting apparatus (110) is identified by means of the image detector (158).

10. Method for treating at least one container (112) for human excretions, by using a cleaning and disinfecting apparatus (110) according to one of the preceding claims, wherein at least one cleaning program is carried out, wherein the container (112) is emptied in the cleaning program, wherein at least one cleaning fluid is applied to the container (112) in at least one cleaning chamber (114), wherein at least one property of the container (112) is identified by means of at least one image detector (158) and wherein the cleaning program is influenced in accordance with the identified property.

11. Method according to the preceding claim, wherein at least one image of the container (112) is recorded by means of the image detector (158).

## Revendications

1. Appareil de nettoyage et de désinfection (110) destiné à traiter au moins un récipient (112) pour excréments humains, comprenant au moins une chambre de nettoyage (114) avec au moins un dispositif à fluide (136) pour soumettre le récipient (112) à au moins un fluide nettoyant, l'appareil de nettoyage et de désinfection (110) étant aménagé pour exécuter au moins un programme de nettoyage, l'appareil de nettoyage et de désinfection (110) comportant au moins un système de commande (146) le système de commande (146) comprenant au moins un dispositif de traitement de données (148), qui par technique programmée est aménagé pour commander le programme de nettoyage, au cours du programme de nettoyage, le récipient (112) étant automatiquement vidé et soumis au fluide nettoyant, l'appareil de nettoyage et de désinfection (110) étant aménagé pour amener le récipient (112) lors d'une vidange d'une position de transport dans laquelle un contenu reste dans le récipient (112) dans au moins une position de vidange, dans laquelle un contenu possible du récipient (112) peut s'écouler hors du récipient (112), le récipient étant pivoté et/ou basculé de telle sorte qu'au moins une ouverture (122) du récipient (112) soit dirigée vers le bas, de sorte que le contenu s'écoule, l'appareil de nettoyage et de désinfection (110) comportant au moins un écoulement (124) avec au moins un siphon (128), l'appareil de nettoyage et de désinfection (110) étant aménagé pour vider le récipient (112), au cours du programme de nettoyage à l'intérieur de l'écoulement (124), l'appareil de nettoyage et de désinfection (110) comportant par ailleurs au moins un détecteur d'images (158), le détecteur d'images (158) étant aménagé pour identifier au moins une propriété du récipient (112) et l'appareil de nettoyage et de désinfection (110) étant aménagé pour influencer le programme de nettoyage en fonction de la propriété identifiée, le détecteur d'images (158) étant placé à l'extérieur de la chambre de nettoyage (114) et étant aménagé pour identifier la propriété avant et/ou pendant une introduction du récipient (112) dans la chambre de nettoyage (114), l'appareil de nettoyage et de désinfection (110) comportant au moins un support (120) destiné à recevoir le récipient (112), le support (120) comportant au moins une position de chargement à l'extérieur de la chambre de nettoyage (114), le récipient (112) pouvant être introduit par un utilisateur dans la position de chargement dans le support (120) et pouvant être retiré du support (120), le support (120) comportant par ailleurs au moins une position de nettoyage à l'intérieur de la chambre de nettoyage (114), dans la position de nettoyage, le récipient (112) étant placé dans la chambre de nettoyage (114) et pouvant être soumis au fluide nettoyant.

2. Appareil de nettoyage et de désinfection (110) selon la revendication précédente, le détecteur d'images (158) comportant au moins une caméra (160).

3. Appareil de nettoyage et de désinfection (110) selon l'une quelconque des revendications précédentes, l'appareil de nettoyage et de désinfection (110) comportant une porte (116) mobile, au moins un support (120) destiné à recevoir le récipient (112) étant relié avec la porte (116), le détecteur d'images (158) étant aménagé pour identifier la propriété du récipient (112) dans le support (120), lorsque la porte (116) est ouverte.

4. Appareil de nettoyage et de désinfection (110) selon l'une quelconque des revendications précédentes, le détecteur d'images (158) étant aménagé pour détecter la propriété dans la position de chargement.

5. Appareil de nettoyage et de désinfection (110) selon l'une quelconque des revendications précédentes, l'appareil de nettoyage et de désinfection (110) étant aménagé par ailleurs pour exécuter au moins un programme d'apprentissage, l'au moins un système de commande (146) de l'appareil de nettoyage et de désinfection (110) étant aménagé par technique de programmation pour permettre ledit programme d'apprentissage, au cours du programme d'apprentissage, un utilisateur introduisant au moins un récipient (112) avec au moins une propriété connue dans une zone visible (164) par le détecteur d'images (158), au moyen du détecteur d'images (158), la propriété étant identifiée et l'utilisateur pouvant prédéfinir une influence du programme de nettoyage correspondant à la propriété et la mémoriser dans l'appareil de nettoyage et de désinfection (110).

6. Appareil de nettoyage et de désinfection (110) selon l'une quelconque des revendications précédentes, l'appareil de nettoyage et de désinfection (110) comportant par ailleurs dans un espace intérieur (118) de la chambre de nettoyage (114) au moins un capteur de processus (174), le capteur de processus (174) étant aménagé pour identifier un niveau d'encrassement du récipient (112), l'appareil de nettoyage et de désinfection (110) étant aménagé pour modifier un déroulement du programme de nettoyage en fonction du niveau d'encrassement identifié.

7. Appareil de nettoyage et de désinfection (110) selon l'une quelconque des revendications précédentes, l'appareil de nettoyage et de désinfection (110) étant aménagé par ailleurs pour identifier, après l'exécution du programme de nettoyage au moins un résultat de nettoyage au moyen du détecteur d'images (158).

8. Appareil de nettoyage et de désinfection (110) selon l'une quelconque des revendications précédentes, l'appareil de nettoyage et de désinfection (110) comportant au moins un dispositif d'avertissement (168) destiné à délivrer au moins un avertissement à un utilisateur.

9. Appareil de nettoyage et de désinfection (110) selon l'une quelconque des revendications précédentes, l'appareil de nettoyage et de désinfection (110) étant aménagé de sorte qu'au moyen du détecteur d'images (158) au moins un objet soit identifié dans au moins une zone à risque de l'appareil de nettoyage et de désinfection (110).

10. Procédé destiné à traiter au moins un récipient (112) pour excréments humains, en utilisant un appareil de nettoyage et de désinfection (110) selon l'une quelconque des revendications précédentes, au moins un programme de nettoyage étant exécuté, pendant le programme de nettoyage, le récipient (112) étant vidé, dans au moins une chambre de nettoyage (114), le récipient (112) étant soumis à au moins un fluide nettoyant, au moyen d'au moins un détecteur d'images (158), au moins une propriété du récipient (112) étant identifiée et le programme de nettoyage étant influencé en fonction de la propriété identifiée.

11. Procédé selon l'une quelconque des revendications précédentes, au moyen du détecteur d'images (158), au moins une image du récipient (112) étant prise.
